# EUROPÄISCHE PATENTANMELDUNG

(11) **EP 3 176 581 A1**
(43) Veröffentlichungstag der Anmeldung: **07.06.2017**
(21) Anmeldenummer: 16202013.5
(22) Anmeldetag: 02.12.2016
(51) Int. Cl.: G01N 33/543

(54) **BAUKASTEN FÜR EIN MULTIPLEX-DRUG DISCOVERY SYSTEM MIT HIGH-THROUGHPUT EIGENSCHAFTEN**

(30) Priorität: 04.12.2015 DE 102015121187
(71) Anmelder: Bundesrepublik Deutschland, vertreten durch den Bundesminister für Wirtschaft und Energie, 12205 Berlin (DE)
(72) Erfinder: JAKUBOWSKI, Norbert, 44319 Dortmund (DE); BRIEL, Andreas, 10245 Berlin (DE); GRUNERT, Bianca, 10245 Berlin (DE)
(74) Vertreter: Zimmermann & Partner Patentanwälte mbB

(57) **Zusammenfassung**

Die vorliegende Erfindung betrifft einen modularen Baukasten zum Erkennen und/oder Identifizieren eines Ziels, zum Beispiel eines Zielmoleküls, einer Zielstruktur oder des modularen Baukastens selbst, wobei der Baukasten einen Container und eine Reportergruppe umfasst, die mit dem Container, vorzugsweise über einen ersten Linker (L1), verbunden ist, wobei die Reportergruppe vorzugsweise konfiguriert ist, um den Container identifizieren zu können.

## Beschreibung

Die Erfindung betrifft ein Baukastensystem, welches in der medizinischen Diagnostik eingesetzt werden kann. Insbesondere betrifft die Erfindung einen modularen Baukasten für ein Multiplex-Drug Discovery System mit High-Throughput Eigenschaften.

Das genetische Material einer Zelle oder eines Organismus wird als Genom bezeichnet. Es liegt in jedem Entwicklungszustand des Organismus unverändert vor. Durch die Möglichkeit der Sequenzierung von Nukleinsäuren und Polymerasekettenreaktion ist es heute möglich, Genome vollständig zu analysieren.

Um komplexe physiologische Vorgänge im Organismus zu verstehen, reicht es jedoch nicht aus, das Genom zu entschlüsseln. Es ist notwendig, die Proteine und andere Biomoleküle in ihrer Gesamtheit zu erfassen. Denn die über die Gene verschlüsselten Proteine, die in einer Zelle oder einem Gewebe exprimiert wurden, sind die eigentlichen biologischen Effektormoleküle. Sie bestimmen das biologische Geschehen, steuern dynamische Prozesse und führen die verschiedensten Funktionen aus.

Die Biosynthese von Proteinen (Translation) ist gewebs- bzw. zellspezifisch. Jedoch sind äußere Bedingungen, wie zum Beispiel Temperatur, Stress, Interaktionen mit anderen Zellen, Medikamente oder Toxine dafür ausschlaggebend, welche Proteine und in welcher Menge diese synthetisiert werden. Das Proteom ist daher ein dynamisches, sich ständig änderndes Gebilde. Nach der Translation können die Proteine durch verschiedene sogenannte posttranslationale Modifikationen weiter verändert werden und so ihre Wirkungsweise ändern.

Die meisten dieser Modifikationen werden durch den Organismus bzw. durch die Zellen selbst ausgelöst. Diese Prozesse können konstitutiv ablaufen oder aber durch Umwelteinflüsse oder andere Parameter beeinflusst werden. Diese Modifikationen sind oft kovalente Bindungen von funktionellen Gruppen an eine oder mehrere Aminosäuren des Proteins.

Zu den posttranslationalen Modifikationen zählen u. a. folgende Vorgänge, die zu neuen Proteinspezies führen: die Abspaltung des N-terminalen Formylrestes von Formylmethionin, die Abspaltung des Methionylrestes am N-Terminus neusynthetisierter Proteine, Fhosphorylierungen, Glykosylierungen, das Knüpfen von Disulfidbrücken, die Veränderung der Faltung durch Chaperone, die gezielte Abspaltung von Signal- oder von anderen Teilsequenzen, die Verknüpfung mit Coenzymen und prosthetischen Gruppen, die Bindung von Ionen und niedermolekularen Substanzen, die Bildung von Proteinkomplexen, die mRNA-unabhängige Synthese von Polypeptiden mit Hilfe von Enzymen, die Proteininaktivierung und -fragmentierung durch Proteolyse, die Ubiquitinierung, die Acetylierung, die Methylierung, die Modifikation mit Fettsäureresten.

Es sind nur etwa 30 posttranslationale Modifikationen bekannt. Ihre wirkliche Anzahl ist jedoch wesentlich grösser. Die Kenntnis dieser Modifikationen ist neben der Identifizierung der zugehörigen Proteine der Schlüssel zum Verständnis der intermolekularen Wechselwirkungen im Organismus.

Es sind zahlreiche Methoden zur Identifizierung und Quantifizierung von Proteinen oder Proteinmischungen bekannt. Diese beschränken sich häufig auf die Identifizierung des Proteins, ohne seine posttranslationalen Modifikationen zu erkennen. Diese sind aber wichtig, um die Reaktionsweisen des Proteins, seinen Aktivitätszustand oder seine Komplexbildung mit anderen Molekülen zu erkennen.

Eine übliche Methode der Proteomanalyse, die es ermöglicht, Proteine aus dem Proteom voneinander zu trennen, ist die 2D-Gelelektrophorese (2D-SDS-PAGE), bekannt aus der Veröffentlichung P. H. O'Farrell, J. Biol. Chem., 1975, 250, 4007-4021. An die Elution der Proteine aus dem Gel schliesst sich die Identifizierung und Charakterisierung der getrennten Spezies an. Die Identifizierung erfolgt zum Beispiel mittels massenspektrometrischer Methoden.

Zahlreiche Proteindatenbanken gestatten dann im Zusammenhang mit Suchkriterien wie der Herkunft des Proteins, seinem Molekulargewicht, der eingesetzten Protease zur Spaltung des Proteins in Peptide die eindeutige Identifizierung des Proteins.

Vor einer 2D-Gelelektrophorese ist häufig die Anreicherung des Proteins zu seiner Messung erforderlich. Außerdem ist dieses Verfahren zeit- und arbeitsaufwendig. Die nicht sehr gute Reproduzierbarkeit des Gels macht es schwierig, zwischen einer Änderung des Elektrophoresesystems und induzierter biologischer Modifikation zu unterscheiden.

Aus M. Uenlue, M. E. Morgan and J. S. Minden, Electrophoresis, 1997, 18, 2071-2077 ist die 2D-Differenz-Gelelektrophorese (DIGE) bekannt. Dabei wird mehr als eine Probe in einem Polyacrylamidgel separiert. Bei dieser Technik müssen zwei Proteinextrakte vormarkiert werden, wobei die Fluoreszenzfarbstoffe cy3 und cy5 eingesetzt werden.

Es ist weiterhin die bioanorganische Analyse von heteroatom- und bevorzugt metallmarkierten Proteinen bekannt. Die Heteroatome in den Proteinen, wie z. B. P, S, Se, Cd und andere Metalle können mit Atomspektroskopie und insbesondere mit LC(liquid chromatography)-gekoppelten ICP(inductively coupled plasma)-MS(mass spectrometry)-Bestimmungen nachgewiesen werden. Weiterhin ist die Detektion mit Laserablations-ICP-MS nach der Trennung des Proteingemisches durch Gelelektrophorese bekannt. Dieses Verfahren diente der Detektion von phosphorylierten Proteinen.

Es ist ferner in der quantitativen Proteomanalyse der Einsatz von isotopen- oder metallcodierten Affinitätstags (ICAT und McCAT) bekannt. Ein Affinitätstag erlaubt die spezifische, reversible Kopplung von Proteinen an ein Trägermaterial. In der anorganischen Massenspektrometrie, insbesondere ICP-MS, sind Markierungen für Verbindungen angewendet worden, die selbst kein detektierbares Heteroelement enthalten. Dabei wurden Alkohole mit Si markiert und Carbonsäuren in pharmazeutischen Proben unter Nutzung von phosphorhaltigen Verbindungen.

Das Markieren von Proteinen durch Metalle und besonders durch Lanthanide und die Detektion durch ICP-MS ist ebenfalls bereits offenbart. Bekannt ist das Markieren von Antikörpern mit Goldnanoclustern in Verbindung mit der Detektion durch Laserablations-ICP-MS auf Blot-Membranen. Fluoreszierende Proben, die Eu, Tb, Dy und Sm enthalten, wurden genutzt, um Antikörper zu markieren und durch ICP-MS in flüssigen Proben zu detektieren.

Die DE 102 27 599 A1 offenbart ein verbessertes CAT-basiertes Verfahren und das entsprechende CAT-Reagenz, das eine Anwendung in der Hochdurchsatzumgebung erlaubt. Das Verfahren dient zur Identifizierung und Quantifizierung von einem oder mehreren Proteinen in einem Proteingemisch mittels eines CAT-Reagenzes vom Typ A-Y-PRG, worin A eine funktionelle Gruppe zur reversiblen, kovalenten oder nichtkovalenten Bindung an ein Trägermaterial darstellt. Y ist eine Gruppe, die mindestens eine Chelatfunktion für isotopenarme Metalle und ein daran gebundenes Metallion umfasst. PRG ist eine reaktive Gruppe zur selektiven Bindung an Peptide oder andere Biomoleküle. Zunächst werden die Proteine der Probe in Peptide gespalten. Dann werden die Peptide an das CAT-Reagenz gebunden. Die markierten Peptide binden an das Trägermaterial und werden so vom Rest der Mischung getrennt. Die Peptide werden eluiert und dann mit Hilfe von Massenspektrometrie nachgewiesen und charakterisiert. In dem Molekül A-Y-PRG wird der Y-Teil u. a. von DOTA (1,4,7,10-Tetraazacyclododekan-1,4,7,10-Tetraessigsäure) und Derivaten davon und DTPA (Diethylentriaminpentaessigsäuredianhydrid) gebildet.

Die US 2005/0059100 A1 offenbart Verbindungen und Methoden, um Biomoleküle zu detektieren, zu analysieren und zu identifizieren. Insbesondere beschreibt die Veröffentlichung elementkodierte Affinitäts-Tags, die ein Metallchelat und ein Metallion beinhalten. Dabei werden zwei Proben mit jeweils unterschiedlichen Tags kontaktiert, wobei die Mengen an Addukt in einer massenspektrometrischen Vergleichsmessung bestimmt werden. Verschiedene Chelatliganden werden vorgeschlagen, darunter auch DOTA- und DTPA-Derivate. Die Bindung der Addukte an Affinitätsmedien wird beschrieben.

Die US 2004/0072250 A1 offenbart Methoden zur Detektion und Messung von elementmarkierten biologisch aktiven Materialien. Zur Detektion der markierten Spezies dienen Atommassen- oder optische Spektrometer mit Atom- oder Atomionenquellen (u. a. Laserablations-ICP-MS). Elementmarkierte Materialien, u. a. Antikörper, Proteinkomplexe und Hormone, die mit dem Elementtag verbunden sind, können in Binding-Assays genutzt werden und durch elementspektroskopische Detektion gemessen werden.

Es werden auch Methoden zur Bestimmung von Metallen in Proben durch Nutzung spezifischer Antikörper zur Isolierung der Zielmetalle beschrieben. Das markierte biologisch aktive Material bildet mit mindestens einem Analyten oder Analytkomplex (ein an andere Moleküle oder biologisch aktive Materialien gebundener Analyt) ein Addukt. Das gebundene markierte Material wird vom ungebundenen Material getrennt und die gebundene markierte Spezies wird mit einem Atommassen- oder optischen Spektrometer detektiert und gemessen. Der Weg zum Erhalt des Adduktes kann die Schritte Markierung, Komplexbildung mit dem Analyten, Abtrennung des gebundenen Materials etc. in verschiedener Reihenfolge enthalten.

Ausgestaltungen der Methoden sehen die Komplexierung von Antikörpern mit Elementspezies (ein an ein anderes Atom oder an eine Atomgruppe gebundenes Element) vor, die dann gemessen werden. Weiterhin wird die Detektion und Quantifizierung eines Analyten durch direktes Markieren des Analyten beschrieben. Eine andere Variante zeigt die Bindung des biologisch aktiven Materials an den Analyten der Probe. Das zu messende Element befindet sich als Tag auf einem sekundären biologisch aktiven Material, das mit dem abgetrennten primären an den Analyten gebundenen biologischen Material kombiniert wird. Der gebildete Komplex wird abgetrennt und vermessen. Letzteres Vorgehen wird erweitert durch die Einführung eines dritten markierten biologisch aktiven Materials, das an einen Komplex aus erstem biologisch aktivem Material, Analyt und zweitem biologisch aktiven Material bindet.

Außerdem werden Kits vorgestellt, die alle nötigen Reagenzien und Anweisungen enthalten, um die oben aufgeführten Methoden durchzuführen. Es wird auch die Möglichkeit eingeschlossen, unterschiedlich markierte biologisch aktive Materialien mit unterschiedlichen Analyten zu komplexieren und so mehrere Analyten simultan zu vermessen. Allen bekannten Methoden gemeinsam ist es, dass jeweils nur eine Komponente des gebildeten Adduktes, das zur Vermessung gelangt, markiert ist. Das Ergebnis der Messung ist jeweils die Identifizierung des Analyten an sich, seine eventuellen Modifikationen werden nicht erkannt.

Die Nutzung der Laserablations-ICP-MS zur Detektion von biologisch aktiven Molekülen ist bekannt. Zu diesem Zweck ist eine optimierte Laserablationszelle für Ablationsmessungen mit der Verwendung von Membranen großer Oberfläche bekannt. Diese Zelle wurde angewendet für den quantitativen Phosphor-Nachweis in phosphorylierten Proteinen.

Alle bekannten Verfahren sind in der Regel aufwendige Bestimmungsmethoden, denen in der Regel komplexe Trennverfahren oder Aufspaltungen der Proteine in Peptide vorausgehen müssen. Oft müssen die Analyten vor der Vermessung angereichert werden.

Häufig ist die Kombination verschiedener Trenn- und Nachweismethoden nötig. Die Simultanbestimmung einer großen Anzahl von Analyten ist nur eingeschränkt möglich.

Insbesondere stellt die Detektion von Heteroelementen - beliebigen chemischen Elementen außer H, C, N und O - mittels Atomspektroskopie ein neues Werkzeug der Biochemie, Molekularbiologie und der Zellbiologie dar. Dabei gewonnene Erkenntnisse über die Verteilung von Metallen in Biomolekülen, Zellen und Geweben münden in das sich allmählich herausbildende Fachgebiet "metallomics" ein.

Die Markierung von Biomolekülen kann prinzipiell auf zweierlei Art - mittels eines labels (labelling) oder mittels eines tags (tagging) erfolgen. Während das bekannteste der labelling-Verfahren, die Markierung mittels Radionuklid, über den Ersatz einzelner Atome durch ein spezifisch nachweisbares Isotop des gleichen Elements sogar unter Beibehaltung der Atomzahl eines Biomoleküls erfolgen kann, setzt das tagging - dem Anbringen eines "Anhängeretiketts" an einem Gepäckstück vergleichbar - stets die Derivatisierung bzw. Modifikation des markierten Biomoleküls voraus. Dabei können spezifische Eigenschaften des Biomoleküls unter Umständen signifikant verändert werden. Die direkte Markierung von Biomolekülen mit chelatisierten Metallen verändert die biologische Aktivität und Spezifität der jeweiligen Biomoleküle erheblich. Bei bisher bekannten Verwendungen von chelatisierten Metallen als tags zur Markierung von Proteinen vor deren Detektion mittels Atomspektroskopie spielt das keine Rolle. Einen Überblick über die Anwendung des Element-gestützten taggings in der Bioanalytik bietet die Publikation von Bettmer, J.; Jakubowski, N. und Prange A. in Analytical and Bioanalytical Chemistry (2006) 386:7-11.

Soll jedoch beispielsweise die Spezifität eines Antikörpers oder die Selektivität eines Enzyms trotz der Modifikation aufrechterhalten werden, scheiden Metallmarkierungen bisher aus. Die vorbekannten Lösungen sind somit nur teilweise zufriedenstellend. Insbesondere sind bekannte Polymertags nur eingeschränkt biokompatibel, weisen eine heterogene Verteilung der Kettenlänge auf und sind damit nur schwer aufzureinigen. Das begrenzt ihre Anwendbarkeit und erschwert insbesondere die Quantifizierung der mit derartigen Markern erzielten Signale. Auf Grund einer nur eingeschränkten Auswahl chelatbildender Gruppen mit hinlänglich hoher Stabilität bzw. Affinität zum Metall, weisen bekannte Marker eine nach wie vor geringe oder gar keine spezifische Empfindlichkeit auf.

Es ist Aufgabe der vorliegenden Erfindung, ein Verfahren der eingangs genannten Art zu schaffen, das einfacher ist und mit dem die simultane Bestimmung von Biomolekülen, insbesondere Proteinen, und deren Modifikationen im Hochdurchsatzverfahren möglich ist.

Die sich daraus ergebenden Aufgaben werden durch den in den Ansprüchen weiter definierten modularen Baukasten und dessen Verwendung gelöst. Weitere Ausführungsformen, Modifikationen und Verbesserungen ergeben sich anhand der folgenden Beschreibung und aus den beigefügten Ansprüchen.

Gemäß einem Aspekt der Erfindung wird ein modularer Baukasten zum Erkennen und/oder Identifizieren eines Ziels, zum Beispiel eines Zielmoleküls, einer Zielstruktur oder des modularen Baukastens selbst bereitgestellt. Der modulare Baukasten umfasst einen Container, und eine Reportergruppe, die mit dem Container, vorzugsweise über einen ersten Linker, verbunden ist, wobei die Reportergruppe vorzugsweise konfiguriert ist, um den Container identifizieren zu können. Der modulare Baukasten umfasst vorzugsweise weiterhin eine Erkennergruppe, die mit dem Container vorzugsweise über einen zweiten Linker (L2) verbunden ist, wobei die Erkennergruppe vorzugsweise konfiguriert ist, um das Ziel, insbesondere das Zielmolekül oder die Zielstruktur, identifizieren zu können.

Gemäß einer Ausführungsform umfasst der modulare Baukasten mindestens eine Füllung, wobei die mindestens eine Füllung sich innerhalb des Containers befindet und wobei die mindestens eine Füllung eine erste Füllung aufweist, die mit dem Container insbesondere über einen dritten Linker verbunden ist, und/oder wobei die mindestens eine Füllung eine zweite Füllung aufweist, die nicht mit dem Container verbunden ist. Vorzugsweise kann mehr als eine erste Füllung und/oder mehr als eine zweite Füllung vorgesehen sein. Die mehreren ersten Füllungen und die mehrere zweiten Füllungen können unterschiedlich sein.

Gemäß einer Ausführungsform weist der Container ein Nanomaterial, wie beispielsweise ein Fulleren, ein Buckminster-Fulleren, einen Buckyball, eine C60-Struktur, ein Nanoröhrchen, ein Vesikel, ein Dendrimer und/oder ein Liposom auf, oder wobei der Container ein Molekül ist, wie beispielsweise ein Chelatbildner, ein Peptid, ein Protein oder ein Antikörper.

Gemäß einer Ausführungsform ist die Reportergruppe ein Atom, insbesondere ein Metall, das zur Detektion mittels Massenspektroskopie geeignet ist, wie beispielsweise eine seltene Erde, und/oder wobei die Reportergruppe ein Molekül ist, das zur Detektion mittels oberflächenverstärkter Raman-Streuung und/oder zur organischen Massenspektroskopie geeignet ist, und/oder wobei die Reportergruppe ein Farbstoff, insbesondere eine Fluoreszenzfarbstoff, ist, der zur Detektion mittels Mikroskopie geeignet ist.

Gemäß einer Ausführungsform weist die Erkennergruppe ein chemisches Molekül auf, das eine hohe Affinität zu dem Zielmolekül oder der Zielstruktur aufweist, wobei die Erkennergruppe typischerweise ein Antikörper, ein Biomolekül, ein Peptid, ein Polypetid, eine RNA, oder eine RNA aufweist.

Gemäß einer Ausführungsform weist die mindestens eine Füllung einen Wirkstoff, einen Botenstoff, einen Inhibitor, einen Katalysator, eine radioaktive Substanz, die vorzugsweise konfiguriert ist, um das Zielmolekül oder die Zielstruktur zu zerstören und/oder bildgebend abzubilden, ein Kontrastmittel beispielsweise für Röntgen oder MRI, und/oder eine Reportergruppe auf.

Gemäß einer Ausführungsform weist der Container ein Schloss auf, mit dem der Container geöffnet werden kann, um seinen Inhalt freizugeben.

Gemäß einem Aspekt der Erfindung wird ein Verfahren zum Erkennen und/oder Identifizieren eines Ziels, zum Beispiel eines biologischen Zielmoleküls oder einer biologischen Zielstruktur, mit einem modularen Baukasten bereitgestellt. Der modulare Baukasten umfasst einen Container, und eine Reportergruppe, die mit dem Container, vorzugsweise über einen ersten Linker, verbunden ist, wobei die Reportergruppe vorzugsweise konfiguriert ist, um den Container identifizieren zu können. Der modulare Baukasten umfasst weiterhin eine Erkennergruppe, die mit dem Container vorzugsweise über einen zweiten Linker verbunden ist, wobei die Erkennergruppe vorzugsweise konfiguriert ist, um das Ziel, insbesondere das Zielmolekül oder die Zielstruktur, identifizieren zu können. Das Verfahren umfasst Kontaktieren des Ziels mit der Erkennergruppe, und Detektieren des modularen Baukastens, wobei der modulare Baukasten vorzugsweise mindestens eine Füllung umfasst, wobei das Verfahren vorzugsweise weiterhin das Freisetzen der mindestens einen Füllung des Containers des modularen Baukastens umfasst.

Gemäß einer Ausführungsform wird das Verfahren zur Exploration von Drug Discovery Systemen für Multiplex-Experimente verwendet.

Gemäß einem Aspekt der Erfindung wird ein Verfahren zur Durchführung simultaner Multiplex-Experimente zur Entwicklung neuer Konstrastmittel durch einen Einsatz von Lanthaniden und/oder Metallen seltener Erden, unter Verwendung eines modularen Baukastens bereitgestellt. Der modulare Baukasten umfasst einen Container, und eine Reportergruppe, die mit dem Container, vorzugsweise über einen ersten Linker, verbunden ist, wobei die Reportergruppe vorzugsweise konfiguriert ist, um den Container identifizieren zu können. Der modulare Baukasten umfasst vorzugsweise weiterhin eine Erkennergruppe, die mit dem Container vorzugsweise über einen zweiten Linker (L2) verbunden ist, wobei die Erkennergruppe vorzugsweise konfiguriert ist, um das Ziel, insbesondere das Zielmolekül oder die Zielstruktur, identifizieren zu können. Der Container des modularen Baukastens weist mindestens eine Füllung auf, wobei die mindestens eine Füllung ein oder mehrere Lanthanide und/oder ein oder mehrere Metalle seltener Erden aufweist. Das Verfahren umfasst Kontaktieren des Ziels mit der Erkennergruppe, Freisetzen der mindestens einen Füllung des Containers, insbesondere der Lanthanide und/oder Metalle seltener Erden, und Nachweis der Lanthanide und/oder Metalle seltener Erden mittels Laser Ablation ICP-MS, insbesondere deren Verteilung.

Gemäß einem Aspekt ist der modulare Baukasten zur Verwendung in der Medizin konfiguriert.

Gemäß einem Aspekt ist der modulare Baukasten zur Verwendung in der medizinischen Diagnostik konfiguriert.

Gemäß einem Aspekt ist der modulare Baukasten zur Verwendung bei der Qualitätskontrolle bei der Herstellung von pharmazeutischen Mitteln, bei der Nachverfolgung der Transportwege von pharmazeutischen Mitteln, und/oder bei der Bestimmung der Verteilung von pharmazeutischen Mitteln in einem biologischen oder medizinischen Objekt, beispielsweise in einem Organismus konfiguriert.

Gemäß einem Aspekt ist der modulare Baukasten zur Herstellung eines Systems zur Verwendung in der Medizin, beispielsweise zur Verwendung in der medizinischen Diagnostik konfiguriert.

Gemäß einem Aspekt ist der modulare Baukasten zur Herstellung eines Systems zur Verwendung bei der Qualitätskontrolle bei der Herstellung von pharmazeutischen Mitteln, bei der Nachverfolgung der Transportwege von pharmazeutischen Mitteln, und/oder bei der Bestimmung der Verteilung von pharmazeutischen Mitteln in einem biologischen oder medizinischen Objekt, beispielsweise in einem Organismus konfiguriert.

Weiterhin sind hierin folgende Systeme, Gegenstände und Verfahren beschrieben, die ebenfalls Aspekte und Ausführungsformen der Erfindung betreffen können und/oder manche Ausführungsformen der Erfindung weiter spezifizieren können. So sollen Merkmale der folgende Systeme, Gegenstände und Verfahren, soweit sie nicht im Widerspruch mit dem soeben offenbarten stehen, mit dem soeben offenbarten kombiniert werden können, um weitere Ausführungsformen der Erfindung zu bilden.

Ebenfalls offenbart ist ein Verfahren mit den Schritten: (a) Immobilisierung von biomolekülerkennenden, das Biomolekül spezifisch bindenden Spezies an definierten Stellen auf einer Oberfläche; (b) Markieren der Biomoleküle mit einem Element-Tag; (c) Kontaktieren der Biomoleküle mit der die biomolekülerkennenden Spezies tragenden Oberfläche; (d) Kontaktieren der an die biomolekülerkennenden Spezies gebundenen Biomoleküle mit modifikationserkennenden Molekülen, welche analytisch differenzierbar sind.

Es versteht sich, dass auch eine andere Reihenfolge der Schritte denkbar ist. Auch versteht sich, dass diese Schritte auch einzeln mit den hierin offenbarten Verfahren kombiniert werden können, um neue Ausführungsformen der Erfindung zu bilden.

Unter dem zu identifizierenden und/oder quantifizierenden Ziel kann insbesondere ein Biomolekül verstanden werden. Unter Biomolekül ist hier jedes Molekül zu verstehen, das biologisch aktiv ist oder sein kann, so zum Beispiel Proteine, Nukleotide, Rezeptoren, Lektine, Oligo- und Polysaccharide, Lipopolysaccharide, Zellmetaboliten, Hormone, pharmakologisch aktive Substanzen, Alkaloide, Steroide, Vitamine, Aminosäuren und Zucker. Die Proteine umfassen u. a. Antigene, Antikörper, Immunoglobuline, Enzyme, Lipoproteine, Glykoproteine, Peptide und Polypeptide. Die Nukleotide umfassen u. a. Oligo- und Polynukleotide, DNA und RNA. Unter den Begriff Biomolekül fallen hier nicht nur natürliche sondern auch synthetisch hergestellte Biomoleküle. Unter synthetisch hergestellten Biomolekülen werden hier Kopien von natürlich vorkommenden Biomolekülen und nicht natürlich vorkommenden Biomolekülen verstanden. Letztere sind zum Beispiel Modellsubstanzen für natürlich vorkommende Biomoleküle, die in der medizinischen Forschung eingesetzt werden. Im Folgenden werden Ausführungsformen beschrieben, die als Ziel beispielhaft ein Biomolekül aufweisen. Die darin dargelegten Prinzipien können jedoch auch auf ein anderes Ziel, insbesondere ein Zielmolekül, das kein Biomolekül ist, und/oder eine Zielstruktur, übertragen werden, ohne von dem Offenbarungsgehalt abzuweichen. So kann anstelle des im Zusammenhang dieser Ausführungsformen beispielhaft beschriebenen Biomoleküls jedes andere geeignete Ziel verwendet werden.

Die Biomoleküle können einzeln vorliegen oder in Mischungen. Sie können aus menschlichen, tierischen oder pflanzlichen Proben, beispielsweise aus Körperflüssigkeiten, Zelllysaten, Pflanzenextrakten, Organsekreten stammen. Es kann sich auch um Fraktionen aus chromatographischen, gelelektrophoretischen oder anderen Trennungen der verschiedensten Biomolekülmischungen handeln.

Unter Element-Tag wird hier eine Markierungsspezies verstanden, die an ein Biomolekül oder eine modifikationserkennende Spezies gebunden werden kann und die ein analytisch differenzierbares Element enthält oder selbst ein solches Element ist. Im Falle von Halogenierungen (z. B. Iodierungen von Histidin- und Tyrosin-Resten in Proteinen) ist das Elementtag ein Halogen-Atom. Element-Tags können auch Atomgruppen oder Moleküle (zum Beispiel Metallkomplexe) sein, die mindestens ein beliebiges analytisch differenzierbares Element enthalten. Insbesondere kann die Erkennergruppe und/oder die Füllung des Containers des erfindungsgemäßen modularen Baukastens ein Element-Tag aufweisen.

Unter biomolekülerkennender Spezies wird jedes Molekül verstanden, das ein zu untersuchendes Biomolekül erkennt und spezifisch bindet. Im bevorzugten Fall, dass es sich bei dem Biomolekül um ein Protein handelt, ist die molekülerkennende Spezies der zugehörige spezifische Antikörper. Insbesondere kann die Erkennergruppe und/oder die Füllung des Containers des erfindungsgemäßen modularen Baukastens eine biomolekülerkennende Spezies aufweisen.

Unter einer Modifikation des Biomoleküls werden hier alle seine funktionellen Gruppen und mit dem Biomolekül verknüpfte Atome, Atomgruppen oder Moleküle verstanden. Modifikationen umfassen zum Beispiel Zuckermoleküle, Metalle, Phosphatgruppen, Ubiquitin, Toxine, Metaboliten verschiedenster Herkunft, Iodierungen. Die Aufzählung ist nicht vollständig. Für den bevorzugten Fall, dass es sich bei dem Biomolekül um Proteine handelt, zählen zu den Modifikationen die posttranslationalen Modifikationen.

Unter modifikationserkennendem Molekül wird jedes Molekül verstanden, das selektiv nur eine bestimmte Modifikation des Biomoleküls erkennt und mit dieser eine feste kovalente oder nichtkovalente Bindung eingeht. In einer bevorzugten Ausgestaltung der Erfindung sind die Biomoleküle Proteine und die biomolekülerkennenden Spezies die zugehörigen Antikörper. Für die Erkennung von Modifikationen in Form von Glykolysierungen kommen als modifikationserkennende Moleküle Lektine in Frage, für die Erkennung von Ubiquitinierungen anti-Ubiquitin, für SUMO(small ubiquitin-related modifier protein)-Anlagerungen anti-SUMO. Das Verfahren ist auf viele denkbare Modifikationen des Biomoleküls übertragbar und lässt das breite Verwendungspotential des modularen Baukastens, der zugehörigen Verfahren und Verwendungen erkennen. Insbesondere kann die Erkennergruppe und/oder die Füllung des Containers des erfindungsgemäßen modularen Baukastens ein modifikationserkennendes Molekül aufweisen.

Im Gegensatz zu einem typischen Vorgehen, bei dem die Immobilisierung von biomolekülerkennenden Spezies auf einer geeigneten Oberfläche vorgesehen ist, ist eine vorherige Immobilisierung nicht unbedingt erforderlich. Eine Immobilisierung kann erfolgen. Es kann jedoch bei der vorliegenden Erfindung der modulare Baukasten auch direkt in ein zu untersuchendes Gewebe und/oder Organismus eingebracht werden. Die zu analysierenden Biomoleküle werden mit Element-Tags markiert und gegebenenfalls mit der Oberfläche kontaktiert. Die biomolekülerkennenden Spezies binden spezifisch die zugehörigen Biomoleküle. In einem weiteren Schritt werden modifikationserkennende Moleküle mit anderen spezifischen, analytisch eindeutig differenzierenren Tags markiert. Es sind auch spezielle Kombinationen ausgewählter, mit differenzierenden Tags versehener modifikationserkennender Moleküle für ein umfassenderes Multiplexing denkbar. Die so vorbereiteten modifikationserkennenden Moleküle werden mit den an die biomolekülerkennende Spezies gebundenen und damit mit gegebenenfalls an der Oberfläche indirekt immobilisierten Biomolekülen in Kontakt gebracht. Ist das Biomolekül Träger der Modifikation, die dem modifikationserkennenden Molekül entspricht, bindet letzteres an die zugehörige Modifikation. Es liegt dann ein System aus molekülerkennender Spezies, Biomolekül mit Modifikation und modifikationserkennender Spezies vor. Die Bestimmung der Identität und die Quantifizierung der vorhandenen Tags ermöglicht die Identifizierung und Quantifizierung des Biomoleküls und seiner Modifikation. Es können auch mehrere Modifikationen gleichzeitig bestimmt werden. In diesem Fall binden mehrere modifikationserkennende Spezies an dasselbe Biomolekül, das Träger verschiedener bzw. mehrfach auftretender identischer Modifikationen ist.

Im Gegensatz zu bekannten Verfahren werden hier keine aufwendigen Trennungsschritte mittels Chromatgraphie oder Elektrophorese benötigt. Die Erfindung nutzt die Erkenntnis, dass sowohl Element-Tags der Biomoleküle als auch analytisch eindeutig differenzierbare modifikationserkennende Moleküle simultan in einem einzigen Detektionsschritt bestimmt werden können.

Ein weiterer Vorteil des modularen Baukastens und der zugehörigen Verfahren und Verwendungen besteht in der Tatsache, dass eine große Vielzahl von Zielen, wie beispielsweise Biomolekülen und ihrer Modifikationen gleichzeitig bestimmt werden kann. Der modulare Baukasten und die zugehörigen Verfahren und Verwendungen gestatten die Identifizierung und Quantifizierung von mehreren Biomolekülen bzw. Biomolekülgemischen und der zugehörigen Modifikationen nebeneinander. Das Verfahren zur gleichzeitigen Bestimmung von Biomolekülen und ihrer Modifikationen ist im Gegensatz zu bekannten Verfahren im Hochdurchsatz durchführbar. Das sogenannte "Multiplexing" zur Darstellung von Unterschieden verschiedener Biomoleküle und/oder Biomolekülgemische ist hier potenziert worden. Es wurde erweitert um die zusätzliche, gleichzeitige Darstellung von Unterschieden zwischen den Arten der Biomoleküle selbst neben der Darstellung von Unterschieden in den Modifikationen innerhalb eines Biomoleküls und der Darstellung von Unterschieden in den Modifikationen mehrerer Biomoleküle im Vergleich zueinander.

Vorzugsweise kann eine Vielzahl von biomolekülerkennenden Spezies vorgesehen sein, die insbesondere auf einer Oberfläche immobilisiert sein können. Das hat den Vorteil, dass der modulare Baukasten und die zugehörigen Verfahren und Verwendungen die gleichzeitige Analyse von einer Vielzahl von Proben und Biomolekülen gestattet. Hochdurchsatzanalysen von komplexen Biomolekülgemischen bei gleichzeitiger Erfassung von Biomolekülmodifikationen sind möglich. Bisher war zum Erhalt dieser Ergebnisse, zum Beispiel der Identifizierung des Proteins und seiner posttranslationalen Modifikationen, eine Kombination von Methoden mit verschiedenen zeit- und arbeitsaufwändigen Arbeitsgängen und Trennverfahren nötig.

Für den Fall, dass eine Immobilisierung auf einer Oberfläche erfolgt, kann die Oberfläche eine Mikroarray-Oberfläche sein. Sie kann aus Metallclustern, Metallen, organischen oder anorganischen Polymeren, Glas oder Halbleitern bestehen. Es sind auch andere Substrate verwendbar. Auf einem Mikroarray bzw. Chip (z.B. im Objektträgerformat 26 mm X 76 mm) können bis zu mehreren Tausend molekülerkennende Spezies an definierten und automatisch ansteuerbaren Positionen, sogenannten Spots immobilisiert werden. Als Oberfläche kann natürlich auch eine Makroarray-Oberfläche verwendet werden.

Biomolekülerkennende Spezies können oft über ihre funktionellen Gruppen direkt auf der Oberfläche verankert werden, indem sie mit reaktiven Gruppen des Oberflächensubstrats kovalente Bindungen eingehen. Andere molekülerkennende Spezies können auch oder nur mit Hilfe von Linkern an die Mikroarrayoberfläche gebunden werden.

Besonders bevorzugt ist insbesondere in der Proteinanalyse eine Goldoberfläche. Die biomolekülerkennenden Spezies im Fall von Proteinen sind in der Regel Antikörper, die mit Hilfe von Linkern auf der Oberfläche des Mikroarrays gebunden werden. Besonders geeignet sind in diesem Zusammenhang thiolgruppenfunktionalisierte Linker, da sie leicht fest auf der Goldoberfläche verankert werden können.

Diese Mikroarrays mit speziell definierten Bindungsstrukturen für zu analysierende Biomoleküle sind kommerziell erhältlich. Sie können vom Fachmann mit den üblichen Techniken aber auch selbst hergestellt werden und mit individuellen biomolekülerkennenden Spezies versehen werden. Die markierten Biomoleküle werden selektiv an die zuvor oberflächenimmobilisierten biomolekülerkennenden Spezies gebunden. Die biomolekülerkennenden Spezies und ihre genauen Positionen auf dem Mikroarray sind bekannt und können zur Detektion der dort gebundenen Biomoleküle gezielt angesteuert werden. Die Kenntnis der angesteuerten biomolekülerkennenden Spezies verbunden mit dem Nachweis des zur Markierung des gebundenen Biomoleküls verwendeten Element-Tags ist ein eindeutiger Hinweis auf die Identität des Biomoleküls.

Der modulare Baukasten und die zugehörigen Verfahren und Verwendungen können auch einen doppelten Beweis für die Identität eines Biomoleküls liefern. Das an seine biomolekülerkennende Spezies gebundene markierte Biomolekül wird dazu erneut mit der biomolekülerkennenden Spezies in Kontakt gebracht, die diesmal mit einem anderen Element-Tag markiert ist als dieses Biomolekül. Das Biomolekül wird sandwichartig zwischen zwei biomolekülerkennenden Spezies eingeklemmt. Die Detektion beider zur Markierung eingesetzten Elemente ist ein eindeutiger Beweis für das Vorhandensein des Biomoleküls, für das die biomolekülerkennende Spezies spezifisch ist. Die doppelte unspezifische Bindung der molekülerkennenden Spezies an ein "falsches" Biomolekül ist sehr unwahrscheinlich und statistisch gesehen auszuschließen. Es liegt ein klarer Beweis für die Identität des Biomoleküls vor.

Durch den modularen Baukasten sowie durch die zugehörigen Verfahren und Verwendungen in Form eines Multiplexing im Mikroformat können in einem Arbeitsgang viele Informationen aus einer Probe gewonnen werden, beispielsweise aus einer auf einem Mikroarray immobilisierten Probe.

Zur eindeutigen Identifizierung des modifikationserkennenden Moleküls in der Analyse kann es analytisch differenzierbare Eigenschaften aufweisen. Ist das Molekül nicht schon von Natur aus Träger derartiger Eigenschaften, bei denen es sich u. a. um enthaltene Heteroatome, chromophore Gruppen oder fluoreszierende Struktureinheiten handeln kann, muss das modifikationserkennende Molekül vor dem Kontaktieren mit den Biomolekülen mit spezifischen, analytisch differenzierbaren Tags markiert werden. Unter spezifischen, analytisch differenzierbaren Tags werden unter anderem Element-Tags verstanden. Dies hat den Vorteil, dass die Elemente dieser Tags gleichzeitig mit den Elementen der Element-Tags der Biomoleküle detektiert werden können. Denkbar ist aber auch zum Beispiel die Einführung von fluoreszierenden oder chromophoren Gruppen.

Vorzugsweise erfolgt die Identifizierung und/oder Quantifizierung des Ziels, wie beispielsweise der Biomoleküle und ihrer Modifikationen, mittels Multielementbestimmung unter Nutzung der Tags und/oder detektierbarer, substanzspezifischer, charakteristischer Strukturmerkmale des Ziels, insbesondere der Biomoleküle oder der modifikationserkennenden Moleküle. Die Strukturmerkmale der Biomoleküle umfassen auch Strukturmerkmale ihrer Modifikationen. Hier weisen der modulare Baukasten und die zugehörigen Verfahren und Verwendungen vorteilhafterweise eine Erweiterung des Multiplexing auf. Es können zusätzlich gleichzeitig Merkmale des Biomoleküls bzw. seiner Modifikationen bestimmt werden, für die keine zusätzliche Markierung nötig ist. In einem einzigen Detektionsschritt können so weitere Informationen gewonnen werden.

In einer Multielementbestimmung werden die Elemente der eingesetzten Element-Tags simultan detektiert. Verfügt das Ziel, wie beispielsweise das Biomolekül, über direkt detektierbare Merkmale, die für eine bestimmte Modifikation charakteristisch sind, können diese neben den Element-Tags direkt eindeutig gemessen werden, was zur Identifizierung der entsprechenden Modifikation führt. Die Detektion und Quantifizierung der spezifischen Tags und damit der entsprechenden zugrundeliegenden Strukturen kann mit Hilfe verschiedener Techniken erfolgen. Für die Detektion können verschiedene Methoden eingesetzt werden, die eine Multielementbestimmung gestatten, wie zum Beispiel organische Massenspektrometrie, anorganische Massenspektrometrie, Atomabsorptionsspektrometrie, ortsaufgelöste LaserAblation in Verbindung mit ICP-MS und ortsaufgelöste Desorptions-Elektrospray-Ionisations-Massenspektrometrie. Die Wahl der Technik richtet sich nach der Art der eingesetzten Tags. Es sind auch Kombinationen aus verschiedenen Messtechniken denkbar. Die Aufzählung hat nur beispielhaften Charakter und beschränkt sich nicht auf die aufgeführten Beispiele. Für die Quantifizierung wird als "innerer Standard" jeweils der Element-Tag des zu analysierenden Biomoleküls bestimmt. Die Messergebnisse werden mit einer entsprechenden Software ausgewertet. Die Auslesung der Signale erfolgt mit verschiedenen Methoden und mit Auflösungen bis zu < 500 µm (z. B. DESI - Desorptions-Elektrospray-Ionisations-(Massen)Spektroskopie, Soft-Landing(Spray)-Desorption der Moleküle bzw. Laserdesorption in Kombination mit organischer oder anorganischer Massenspektrometrie). Die Techniken können alleine oder in Kombination eingesetzt werden.

Besonders bevorzugt unter den Multielementdetektionstechniken ist die Laserablation in Verbindung mit ICP-MS. Hier wird ein definierter Abtrag von Material aus einer Probe mittels eines fokussierten Laserstrahles in einer Laserablationskammer genutzt. Diese ist mit einem Massenspektrometer mit einer induktiv gekoppelten Plasmaionenquelle zum Nachweis der im Probenmaterial enthaltenen Bestandteile gekoppelt. Das abgetragene Probenmaterial wird mit einem Transportgas (meist Argon) in die Plasmaionenquelle des ICP-MS transportiert und dort weiter atomisiert und ionisiert. Die LA-ICP-MS hat gegenüber anderen Multielementbestimmungsmethoden zahlreiche Vorteile. Sie ist schnell, quantitativ und auch im Spurenbereich einsetzbar. Sie gibt keinerlei Einschränkungen bezüglich der physikalischen oder chemischen Eigenschaften des Probenmaterials. Es sind nahezu alle Elemente auch quantitativ mit hoher Prazision zu bestimmen. Ihr großes Potential für die Proteinforschung ist bereits bekannt.

Ist die Modifikation ein an eine funktionelle Gruppe des Biomoleküls gebundener medizinischer Wirkstoff, zum Beispiel das Cytostatikum cis-Platin aus der Krebsbehandlung, kann das Platin direkt nachgewiesen werden. In diesem und anderen ähnlich gelagerten Beispielen kann erkannt werden, an welches Protein der Wirkstoff bzw. seine platinhaltigen Metaboliten binden. Dies gibt Hinweise auf die Wirkungsweise des Wirkstoffes.

Die Erfindung schließt nicht aus, dass das Biomolekül selbst oder die am Biomolekül befindlichen Modifikationen bereits Träger einer "natürlichen" Markierung sind. Derartige substanzspezifische Eigenstrukturen und/oder -merkmale der zu analysierenden Biomoleküle oder ihrer Modifikationen können zum Beispiel Chromophore, fluoreszierende Gruppen, bestimmte chemische Elemente oder ihre Isotope sein. Sie können gemeinsam mit elementmarkierten Modifikationen und Biomolekülen identifiziert und quantifiziert werden. Dazu zählt beispielsweise die Phosphorylierung. Der Nachweis von Phosphor spricht für eine am Protein befindliche Phosphatgruppe.

Die Bestimmung der Identität und Quantifizierung der Biomoleküle durch Multielementbestimmung erfolgt für diese Fälle in Kombination mit anderen Bestimmungsmethoden, wenn das analytisch differenzierbare Strukturmerkmal kein charakteristisches Element ist, sondern als zum Beispiel chromophore oder fluoreszierende Gruppe anderen Analysetechniken zugänglich ist. Bevorzugterweise erfolgt die Elementbestimmung und/oder -quantifizierung dann durch eine Kombination mit Fluoreszenzspektrophotometrie, UV/VIS-Spektrophotometrie oder anderen geeigneten Detektionstechniken. Die Erfindung hat den Vorteil, dass sie in einem Arbeitsgang nicht nur die Elementbestimmung gestattet; sondern auch die Bestimmung anderer charakteristischer Merkmale zulässt.

Insbesondere vorteilhaft für die eindeutige Identifizierung eines Biomoleküls und seiner Modifikationen ist, wenn sich die an den modifikationserkennenden Molekülen detektierbaren Elemente von den Elementen des Tags des modifikationstragenden Biomoleküls unterscheiden.

Das Biomolekül kann in einer Probe vorliegen, dies einzeln oder im Gemisch. Sollen die Zusammensetzungen mehrerer unterschiedlicher biomolekülhaltiger Proben nebeneinander untersucht werden, werden die Biomoleküle jeder Probe jeweils mit unterschiedlichen Element-Tags markiert. Liegen in den Proben identische Biomoleküle vor, die an die gleiche biomolekülerkennende Spezies gebunden werden, können sie somit anhand der eingesetzten Elemente unterschieden werden. Der Nachweis des entsprechenden Elements ordnet das Biomolekül eindeutig der Herkunftsprobe zu.

Die Modifikationen des Biomoleküls können durch modifikationserkennende Moleküle erkannt werden, die mit den Modifikationen eine feste Bindung eingehen. In einer Ausgestaltung erkennen derartige modifikationserkennende Moleküle am Biomolekül vorhandene funktionelle Gruppen und binden diese spezifisch. In einer bevorzugten Ausgestaltung erkennen modifikationserkennende Moleküle posttranslationale Modifikationen und binden diese spezifisch. Dies ist der Fall, wenn es sich bei dem Biomolekül um ein Protein handelt. Das Verfahren erweitert damit deutlich die Möglichkeiten in der Proteomanalyse.

In einer weiteren Ausgestaltung erkennen und binden modifikationserkennende Moleküle spezifisch an das Biomolekül gebundene Moleküle, Atome oder Atomgruppen. Dabei kann es sich zum Beispiel um Adduktbildungen des Biomoleküls mit Pharmaka, Toxinen, Metabolismusprodukten des Stoffwechsels, Nahrungsmittelbestandteilen oder deren Abbauprodukten und mit Allergenen handeln.

In bevorzugten Ausgestaltungen sind die biomolekülerkennende Spezies und das modifikationserkennende Molekül ebenfalls ein Biomolekül. Derartige Biomoleküle sind bereits in großer Zahl beschrieben worden. Sie sind oft kommerziell erhältlich, oder es sind für den Fachmann leicht nachvollziehbare Verfahren zu ihrer Herstellung beschrieben worden. In der Proteinanalyse handelt es sich bei den biomolekülerkennenden Spezies vorzugsweise um Antikörper, bei den modifikationserkennenden Molekülen kann es sich um Lektine, anti-Ubiquitin oder anti-SUMO handeln.

Beispielsweise nach der Probenvorbereitung, die sich nach der Art der Probe richtet und vom Fachmann unter Kenntnis der entsprechenden Literatur leicht durchgeführt werden kann, können die Biomoleküle mit ausgewählten Element-Tags markiert werden. Diese Art der Markierung hat den Vorteil, dass als Elemente fast alle Elemente des Periodensystems in Frage kommen, ausgenommen Wasserstoff und die Edelgase. Weiterhin von Vorteil ist, dass sich im bevorzugten Fall der Proteinmarkierung viele funktionelle Gruppen innerhalb der Polypeptidkette anbieten, an die ein Element-Tag leicht binden kann (zum Beispiel Hydroxylgruppen, Aminogruppen, Thiolgruppen). Derartige Markierungen sind bereits offenbart. Oft kann auf kommerziell erhältliche Element-Tags zurückgegriffen werden. Sie können auch leicht nach bekannten Methoden hergestellt werden.

Es ist von Vorteil, wenn die für die Markierung der Biomoleküle und/oder der modifikationserkennenden Moleküle eingesetzten Elemente der Element-Tags Metalle sind. Es können aber auch alle anderen Elemente des Periodensystems und ihre Isotope verwendet werden, ausgenommen Wasserstoff und Edelgase. Die meisten Metalle sind in der Regel nicht in Biomolekülen oder ihren Modifikationen enthalten, so dass ihr Nachweis bei der Detektion ein eindeutiger Hinweis auf ihre Herkunft aus dem Element-Tag ist. Metalle sind der Multielementdetektion leicht zugänglich. Sie sind einfach zu chelatisieren und über funktionalisierte Chelate gezielt fest an funktionelle Gruppen des Biomoleküls zu binden. Diese Markierungen gehören zum Stand der Technik. Die Tags verändern das Biomolekül und seine Modifikationen selbst nicht.

Besonders bevorzugt sind Lanthanide, für die zahlreiche stabile Chelatkomplexe beschrieben sind, die als Element-Tag geeignet sind. In einer besonders bevorzugten Ausgestaltung besteht der Chelatkomplex aus einem Lanthanidmetall und einem Liganden vom Typ DTPA, DTPA-Derivat, DOTA oder DOTA-Derivat. Für diese Komplexe sind in freier oder gebundener Form Stabilitätskonstanten für verschiedene Metalle beschrieben worden. Diese Konstanten belegen, dass Metallaustauschreaktionen mit anderen Metall-ChelatKomplexen nicht messbar erfolgen. Wird folglich das zur Markierung des Biomoleküls verwendete Metall detektiert, ist dies ein eindeutiger Beweis für das Vorhandensein eines Biomoleküls, das gezielt mit diesem Komplex markiert wurde.

Der modulare Baukasten und die zugehörigen Verfahren können Verwendung zur Genomanalyse, zur Metabolitenanalyse, in der Rezeptorforschung, zum Drogen-Screening, in der Cell-on-the-Chip-Technologie, zur Computer-Simulation von zellulären Vorgängen, in der Systembiologie, zur Untersuchung von biomolekularen Interaktionen und der endokrinen Disruption (Modulation hormoneller Rezeptoren durch Umweltgifte), in der Toxikologie, in der Umweltanalytik, in der Pharmakologie, zur Entwicklung neuer Arzneimittel, zur medizinischen Diagnostik, zur Vorsorge und zum Screening in der Krebsdiagnostik, zur Untersuchung einer von der Pflanze, dem Tier oder dem Menschen entnommenen und Biomoleküle enthaltenden Probe und/oder allgemein in der biochemischen Forschung finden.

Ein weiterer Aspekt sind das Design und die Zusammenstellung kompatibler Detektionsreagenzien, zum Beispiel in Form eines Kits, das in dem Verfahren zur Multiplexbestimmung von Biomolekülen und ihrer Modifikationen verwendet werden kann. Eine Ausgestaltung sieht vor, für ausgewählte Anwendungen ein derartiges Kit bereitzustellen. Es kann beispielsweise zur Bestimmung des Glykolysierungsgrades eines bestimmten Proteins dienen. Dieses in dem Verfahren verwendbare diagnostische Kit zur Identifizierung und Quantifizierung von Biomolekülen kann folgende Komponenten umfassen: (a) ein mit biomolekülerkennenden Spezies versehenes Mikroarray oder ein Mikroarray und auf dem Mikroarray zu fixierende biomolekülerkennende Spezies; (b) Markierungsreagenzien zur Markierung von Biomolekülen; (c) modifikationserkennende Spezies und Markierungsreagenzien zur Markierung der modifikationserkennenden Spezies oder bereits markierte modifikationserkennende Spezies. Insbesondere kann das diagnostische Kit den modularen Baukasten aufweisen.

In einer weiteren Ausgestaltung umfasst das diagnostische Kit ferner einen Linker zur Fixierung der biomolekülerkennenden Spezies auf dem Mikroarray. Besonders bevorzugt ist die Verwendung des diagnostischen Kits zur Proteinanalyse.

Außerdem denkbar ist die Verwendung des diagnostischen Kits zur Genom- und Metabolitenanalyse, zur Rezeptorforschung, zum Drogen-Screening, zur "Cell on the Chip"-Technologie, zur Computer-Simulation von zellulären Vorgängen, in der Systembiologie, zur Untersuchung bimolekularer Interaktionen und der endokrinen Disruption (Modulation hormoneller Rezeptoren durch Umweltgifte), in der Toxikologie, in der Umweltanalytik, in der Pharmakologie, zur Entwicklung neuer Arzneimittel, zur medizinischen Diagnostik, zur Vorsorge und zum Screening in der Krebsdiagnostik, zur Untersuchung einer von der Pflanze, dem Tier oder dem Menschen entnommenen und Biomoleküle enthaltenden Probe und/oder allgemein in der biochemischen Forschung.

Es wurde ein analytisches Verfahren entwickelt wurde, welches Biomoleküle und seine Modifikationen simultan identifiziert, quantifiziert und charakterisiert und welches im Hochdurchsatzverfahren einsetzbar ist. Dies ist gleichzeitig für zahlreiche Proben, Probengemische und deren Bestandteile möglich.

Es ist im Mikromaßstab durchführbar und tauglich für den Mikroarray-Massstab. Es werden komplexe Analysendaten mit hohem Informationsgehalt hinsichtlich vorhandener Biomoleküle und abgewandelter Biomolekülstrukturen (Modifikationen) zur Verfügung gestellt. Grundlage können Mikroarrays mit speziell definierten Bindungsstrukturen für zu analysierende Moleküle sein. Insbesondere können spezielle Kombinationen ausgewählter, mit differenzierenden Tags versehener Erkennungsmoleküle für ein Multiplexing vorgesehen sein, das eine simultane Identifizierung und Quantifizierung von Biomolekülen und ihrer verschiedenen Modifikationen erlaubt.

Ferner wird eine Markersubstanz mit einer reaktiven Gruppe vorgeschlagen, wobei die Markersubstanz für den spezifischen Nachweis von Biomaterial angepasst ist und ein Trägermaterial umfasst, das zumindest zwei Stück einer Untereinheit enthält, die miteinander über eine Peptidbindung verknüpft sind, wobei die Untereinheit zumindest eine Aminosäure mit einer Heteroelement-haltigen Reportergruppe aufweist, und das Heteroelement ein beliebiges chemisches Element außer Wasserstoff (H), Kohlenstoff (C), Stickstoff (N) und Sauerstoff (O) ist. Insbesondere kann der modulare Baukasten, insbesondere die Füllung des Containers des modularen Baukastens, die Markersubstanz aufweisen. Auch kann, insbesondere dann wenn die Markersubstanz eine Reportergruppe aufweist, die Markersubstanz mit dem Container verbunden sein und/oder vorzugsweise konfiguriert sein, den Container identifizieren zu können. Vorteile dieser Ausführungsform bestehen in der ausgesprochen guten Bio-Verträglichkeit der resultierenden Markersubstanz sowie in der Möglichkeit, über unterschiedliche, jeweils an die Untereinheit gebundene Heteroelemente mittels atomspektroskopischer Analysemethoden ein für das Lanthanoid-dekorierte Peptid charakteristisches Signalmuster zu erzeugen. Das einer (poly-) peptidischen Grundstruktur, d.h. der Trägersubstanz, zuzuordnende Signalmuster setzt sich beispielsweise aus den Atomspektren oder den Massenzahlen der anwesenden Elemente zusammen. Da das anteilige Masse-Verhältnis der Elemente in gleichmäßig mit Reportergruppen versehenen Peptiden konstant ist, kann über die Anzahl der über Peptidbindungen miteinander verknüpften Untereinheiten und somit über die Länge des Polypeptids die erzielte Signalstärke einer gewünschten Signalstärke bzw. der jeweiligen Messsituation angepasst werden.

Ferner wird eine Markersubstanz vorgeschlagen, deren Untereinheit selbst ein Peptid, insbesondere ein Polypeptid ist. Gemäß einer Modifikation dieser Ausführungsform können verschiedene Untereinheiten, also verschiedene Peptide vorliegen, die über Peptidbindungen miteinander verknüpft sind. Besondere Vorteile dieser Ausführungsform ergeben sich daraus, dass ein bestimmtes Masse-Verhältnis der vorliegenden Heteroelemente zur Unterscheidung unterschiedlicher Markersubstanzen und der mit ihnen markierten Targets, wie beispielswiese den Container des modularen Baukastens, verwendet werden kann, indem Peptide als Untereinheit genutzt werden, die aus unterschiedlichen Aminosäuren, welche wiederum mit unterschiedlichen Metallen befrachtet sind, zu einem Polypeptidstrang miteinander verknüpft werden können. So kann die Markersubstanz eine kodierte Nachricht tragen oder zur spezifischen Kennzeichnung eines Targets, wie beispielswiese des Containers des modularen Baukastens, verwendet werden.

Insbesondere kann die Reportergruppe und/oder die Markersubstanz zur Identifizierung und/oder Kennzeichnung des Containers verwendet werden. Insbesondere dann, wenn die Reportergruppe mit dem Container, beispielsweise über den ersten Linker, verbunden ist, und/oder die Reportergruppe an einer Außenseite des Containers vorgesehen ist. Eine "Außenseite des Containers" kann in dem Zusammenhang als die Seite des Containers verstanden werden, in dem eine Füllung bei geschlossenem Zustand des Containers nicht vorgesehen ist. Ist die Reportgruppe jedoch als eine Füllung des Containers vorgesehen, so kann die Reportergruppe zur Identifizierung und/oder Kennzeichnung eines, insbesondere anderen, Targets verwendet werden, wie beispielsweise eines Zielmoleküls und/oder einer Zielstruktur wie sie hierin beschrieben sind.

Ebenso kann die Spezifität und Selektivität des Targets genutzt werden, um unterschiedlichste molekulare und/oder zelluläre und/oder sub-zelluläre Strukturen zu identifizieren und/oder zu quantifizieren.

Ferner können ebenso verschiedene Peptide die mit verschiedenen Metallionen befrachtet sind in einem Polypeptidstrang miteinander verbunden vorliegen. Genauso ist es möglich, Chelatbildner unterschiedlicher Affinität für unterschiedliche Metallionen zur Markierung verschiedener Aminosäure-Seitenketten eines Peptidstrangs zu nutzen. Ziel und Vorteil der dem Fachmann an Hand der hier gegebenen Informationen geläufigen Maßnahmen ist es, ein (vor)bestimmtes Verhältnis verschiedener Heteroelemente zu erreichen, das sich - nach einer entsprechenden Kalibrierung - mittels atomspektroskopischer Methoden nachweisen und zur Identifikation einer Markierung in einer unbekannten Probenzusammensetzung verwenden lässt.

Ferner kann die Reportergruppe des erfindungsgemäßen modularen Baukastens zu diesem Zweck ein Heteroelement aufweisen, das ausgewählt ist unter allen Metallen, die im untersuchten Biosystem nicht vorkommen. Insbesondere ist das Heteroelement ausgewählt unter: Lanthan (La), Cer (Ce), Praseodym (Pr), Neodym (Nd), Promethium (Pm), Samarium (Sm), Europium (Eu), Gadolinium (Gd), Terbium (Tb), Dysprosium (Dy), Holmium (Ho), Erbium (Er), Thulium (Tm), Ytterbium (Yb), Lutetium (Lu), Silber (Ag), Aluminium (Al), Arsen (As), Gold (Au), Beryllium (Be), Wismut (Bi), Cadmium (Cd), Germanium (Ge), Kobalt (Co), Chrom (Cr), Kupfer (Cu), Eisen (Fe), Gallium (Ga), Quecksilber (Hg), Indium (In), Lithium (Li), Mangan (Mn), Natrium (Na), Blei (Pb), Rubidium (Rb), Antimon (Sb), Skandium (Sc), Selen (Se), Zinn (Sn), Tellur (Tl), Vanadium (V), Wolfram (W), Yttrium (Y), Zink (Zn), Zirkonium (Zr), Schwefel (S), Phosphor (P), Silizium (Si), Iod (I), Brom (Br), Fluor (F).

Weiterhin können auch Metallnanopartikel, -Cluster oder -Quantenpunkte dieser Elemente in der Reportergruppe des erfindungsgemäßen modularen Baukastens verwendet werden, ebenso Nichtmetalle, wie Schwefel (S), Phosphor (P), Silizium (Si) (z.B. in Form von MSTFA (N-methy-N-(trimethylsilyl) trifluoroacetamid), Iod (I), Brom (Br) und Fluor (F) oder eines oder mehrere dieser Elemente enthaltende Nanopartikel.

Ein Vorteil dieser Ausführungsform ergibt sich beispielsweise daraus, dass mit Lanthanoiden (Lanthaniden) sich chemisch nahezu identisch verhaltende Elemente zur Verfügung stehen, die sich deutlich von den in biologischen Matrices anzutreffenden chemischen Elementen unterscheiden. Daraus ergeben sich Vorteile für den ungestörten und eindeutigen Nachweis der Markersubstanz und der mit ihr modifizierten Targets, wie beispielswiese den Container des modularen Baukastens.

Ferner wird eine Reportergruppe, insbesondere als Teil des modularen Baukastens und/oder als Teil der Markersubstanz und dadurch als Teil des modularen Baukastens, vorgeschlagen, die einen Chelatbildner, einen Komplexbildner und/oder einen Ionenaustauscher umfasst. Vorteile ergeben sich aus der Möglichkeit verschiedene, ggf. auch nicht der Gruppe der Lanthanoide zuzurechnende Elemente an die Reportergruppe und damit gegebenenfalls an die Markersubstanz, insbesondere jedoch an den modularen Baukasten, binden zu können. In Abhängigkeit von der jeweils anzutreffenden Messsituation kann das zu Nachweis verwendete Heteroelement, also ein chemisches Element, das in der jeweiligen unmarkierten Probe nicht oder nur in vernachlässigbarer Konzentration vorkommt, mit größerer Freiheit gewählt werden. Weiterhin können Metalle genutzt werden, um über einen geeigneten Chelatliganden eine Fluorophorgruppe anzudocken.

Ferner wird eine derartige Markersubstanz vorgeschlagen, deren Chelat- und/oder Komplexbildner ausgewählt ist unter: Cyclohexandiamintetraessigsäure (CDTA); Diethylentriaminpentaessigsäure (DTPA); Ethylendiamintetraessigsäure (EDTA); Ethylenglycol-bis(aminoethylether)-N,N,N',N'-tetraessigsäure (EGTA); Nitrilotriessigsäure (NTA), N-(2-Hydroxyethyl)-ethylendiamin-N,N,N'-triessigsäure (HEDTA); Triethylentetraminhexaessigsäure (TTHA) oder 1,4,7,10-Tetraazacyclododecan-1,4,7,10-tetraessigsäure (DOTA) oder DOTA-Derivate.

Vorteile ergeben sich aus der kommerziellen Verfügbarkeit der benannten Chelatbildner, ihrer guten Wasserlöslichkeit und der, insbesondere für DOTA und für DTPA ausgeprägte Stabilität der erhaltenen Komplexe. Beide letztbenannten Komplexe sind achtzähnig und komplexieren das dreiwertige Kation besonders stabil.

Ebenso kann ein Chelatbildner einer Reportergruppe des erfindungsgemäßen modularen Baukastens ausgewählt sein unter: 1,4, 7-Triazacyclononan-1,4,7-triessigsäure (NOTA), NOTA-Derivat, 1,4,8,11-Tetraazacyclotetradecan-1,4,8,11-tetraessigsäure (TETA), Diethylentriamin-N,N',N"-triessigsäure-N,N"-bis(2-methylpyridin) (DTPA-BP), Diethylentriamin-N,N',N"-triessigsäure-N,N"-bis(methylamid) (DTPA-BMA), 1-Oxa-4, 7,10-tetraazacyclododecane-4,7,10-triessigsäure (DO3A), DO3A-Derivat, Diethylentriamin-N,N,N',N",N"-pentaessigsäure (DTPAA), p-Isothiocyanatobenzyl-desferrioxamine (Df-Bn-NCS), 1,4,7,10,13,16,19,22-Oktaazacyclotetracosan-1,4,7,10,13,16,19,22-oktaessigsäure (OTEC), 1,4,7,10,14,17,20,23-Oktaazacyclohexacosan-1,4,7,10,14,17,20,23-oktaessigsäure (OHEC), 1,4,7,10-Tetraazacyclododecane-1,4,7,10-tetra(methylene phosphonate) (DOTP), benzyloxypropionictetraacetate (BOPTA); Calixarene; Cryptate, z.B. trispyridine europium cryptate (TBP cryptate); 1,4,8,11-tetraazacyclotetradecane (cyclam); 1,4,7,10-tetraazacyclododecane (cyclen); 1,4,7-triazacyclononane (tacn), oder unter Carbonyl-Komplexen, Carben-Komplexen und Metallocen basierten Reagenzien.

Ferner kann die Verknüpfung der Reportergruppe, insbesondere zum Container des modularen Baukastens, zum Beispiel der erste Linker, und/oder zum Trägermaterial, eine Thiocarbonyl-Gruppe oder eine Thioether-Bindung aufweist. Die Reportergruppe ist jeweils kovalent mit der Seitenkette einer Aminosäure verknüpft. Die kovalente chemische Bindung kann auf unterschiedliche Art und Weise bewerkstelligt werden, wobei bevorzugte Umsetzungen unter milden Reaktionsbedingungen ablaufen. Unter milden Reaktionsbedingungen wird beispielsweise verstanden: Temperatur nicht wesentlich oberhalb von 40 °C, pH-Werte nicht oder nur kurzzeitig außerhalb des physiologischen Bereichs von pH 6 bis pH 9, insbesondere bevorzugt um pH 7. Unter diesen Bedingungen kann eine Markierung des jeweiligen Targets, wie beispielswiese des Containers des modularen Baukastens, vorgenommen werden, ohne dass das Target während der Markierung seine Spezifität vermindert oder verliert. Das ist insbesondere wichtig, wenn das Target ein Antikörper, d.h. ein Immunoglobulin oder ein Antikörperfragment, beispielsweise Fab, ist.

Ferner kann die Reportergruppe über eine Seitengruppe einer Aminosäure mit dem Container des modularen Baukastens und/oder der Trägersubstanz der Markersubstanz verbunden sein. Das bietet den Vorteil über unterschiedliche, den jeweiligen Seitengruppen angepasste, Reaktionsbedingungen verschiedene chemische Elemente an einem Peptid verankern zu können. Auf diese Art und Weise ergibt sich die Möglichkeit, ein bestimmtes Masseverhältnis unterschiedlicher Elemente einzustellen, das für das jeweilige Polypeptid bzw. den resultierenden modularen Baukasten oder die resultierende Markersubstanz charakteristisch ist. Daraus ergeben sich die eingangs erläuterten Möglichkeiten einer spezifischen Kodierung.

Ferner wird vorgeschlagen, dass die zur Bindung von Reportergruppen genutzten Seitenketten ausgewählt sind unter Seitengruppen der Aminosäuren Arginin, Cystein, Histidin, Hydroxylysin, Lysin und/oder Serin.

Ferner werden für die hier beschriebenen Trägermoleküle die nachfolgenden, mit einem gebräuchlichen Kürzel und dem entsprechenden Trivialnamen aufgeführten Aminosäuren verwendet: Ala (Alanin); Arg (Arginin); Asn (Asparagin); Asp (Asparaginsäure); Cys (Cystein); Glu (Glutaminsäure); Gln (Glutamin); Gly (Glycin); His (Histamin); HyLys (Hydroxylysin); HyPro (Hydroxyprolin); Ileu (Isoleucin); Leu (Leucin); Lys (Lysin); Met (Methionin); Phe (Phenylalanin); Pro (Prolin); Ser (Serin); Thr (Threonin); Try (Tryptophan); Tyr (Tyrosin); Val (Valin). Dabei stellen Cystein, Methionin und Selenocystein eine besondere Gruppe dar, da diese bereits ein Reporterelement enthalten (S bzw. Se), welches zum Nachweis herangezogen werden kann.

Ferner können ausdrücklich nicht ausschließlich proteinogene Aminosäuren zum Aufbau der Trägersubstanz verwendet werden. Beispiele dieser zum Aufbau geeigneter Trägermoleküle gleichwertig einsetzbarer Aminosäuren sind Ornithin, Citrullin, DOPA, Homoserin oder Thyroxin. Dem Fachmann sind weitere Aminosäuren bekannt. Dabei ist die optische Aktivität der Aminosäure bzw. deren Reinheit hinsichtlich des Gehalts an D- und L-Formen der jeweiligen Aminosäure für die vorgeschlagene Anwendung nicht entscheidend.

Vorteile der Verwendung dieser Aminosäuren ergeben sich daraus, dass die funktionellen Gruppen der Seitenketten dieser Aminosäuren chemisch deutlich voneinander verschieden sind, so dass über die Wahl der jeweiligen Kopplungsreagenzien, des jeweiligen pH-Wertes, der jeweiligen Ionenstärke und/oder der Temperatur ausschließlich eine Aminosäure mit einem spezifischen Element markiert werden kann. Nachfolgend kann dann eine andere Aminosäure mit einem Metall-Ion dekoriert werden, danach die nächste, und so fort, bis das gewünschte Element-Verhältnis eingestellt ist.

Ferner wird eine Markersubstanz vorgeschlagen, deren Trägermolekül ein Peptid, umfassend eine Sequenz von D- und/oder L-Aminosäuren umfasst, wobei die Zahl der das Peptid ausbildenden Aminosäuren ausgewählt ist unter 2 bis 150, beispielsweise unter 3 bis 100, insbesondere unter 3 bis 50 und typischerweise zwischen 4 bis 36. Vorteile dieser Ausführungsform ergeben sich daraus, dass auch Racemate, also Enantiomeren-Mischungen, wie sie für synthetisch hergestellte Aminosäuren typisch sein können, verwendbar sind und die erhaltene Kettenlänge eine hinlänglich gute Löslichkeit des darauf basierenden Markermoleküls besitzt. Zudem ist die Trennung von Racematen zumeist aufwendig und kann für verschiedene Anwendungen der vorgeschlagenen Markersubstanz unterbleiben. Die erhaltene Markersubstanz ist damit kostengünstiger herstellbar.

Ferner kann die vorgeschlagene Markersubstanz eine einzige lineare Sequenz von Aminosäuren darstellen. Vorteile ergeben sich aus der überschaubaren Möglichkeit der chemischen Modifizierung derartiger Moleküle.

Ferner kann die Markersubstanz ein Trägermolekül aufweisen, das ein zyklisches Peptid ist. Vorteile ergeben sich aus den zusätzlichen, ggf. vorteilhaften Eigenschaften eines Cyclopeptids. Beispielsweise kann die Löslichkeit eines Cyclopeptids in einem gegebenen Lösungsmittel von jener eines linearen Peptids einer identischen Zusammensetzung abweichen, oder eine physiologische Wirkung eines Cyclopeptids kann von jener eines linearen Peptids abweichen. Weitere Vorteile ergeben sich aus den spezifischen Eigenschaften des jeweils gewählten cyklischen Peptids. Ebenso kann die Stabilität eines zyklischen Peptids über oder unter jener eines linearen Peptids gleicher Zusammensetzung liegen.

Ferner wird eine Markersubstanz vorgeschlagen, die zumindest eine halogenierte Aminosäure aufweist. Insbesondere ist eine Seitengruppe zumindest eines Typs der im Trägermolekül vorhandenen Aminosäuren halogeniert. Die Besonderheiten von Halogenen, insbesondere bezüglich ihres Nachweises an Hand spezifischer Eigenschaften vervielfachen die Möglichkeiten der bereits erläuterten Kodierung und können zur Steigerung der Nachweisempfindlichkeit von Analysemethoden genutzt werden, die die beschriebenen Markersubstanzen nutzen.

Ferner können die zur Halogenierung genutzten Elemente Brom, Chlor, Jod und/oder Fluor sein. An diese Elemente angepasste Nachweisverfahren, sowie die mit Hilfe dieser Elemente zusätzlich möglichen Verhältnisse der beispielsweise bei der Massenspektrometrie auftretenden Massezahlen eröffnen weitere Möglichkeiten der spezifischen Markierung und/oder Kodierung sowie zur Steigerung der Nachweisempfindlichkeit und der Spezifität von Analysen.

Ferner wird ein Verfahren zur Herstellung einer Markersubstanz vorgeschlagen, das die folgenden Schritte umfasst: a) Auswählen eines Moleküls mit wenigstens einer terminalen funktionellen Gruppe, umfassend miteinander zu einem Peptid verknüpfte Aminosäuren, wobei wenigstens eine Aminosäure eine Seitenkette aufweist und wobei die terminale funktionelle Gruppe ausgewählt ist unter einer Aminogruppe und einer Carboxylgruppe; b) Umsetzen wenigstens einer Seitenkette einer Aminosäure mit einem bifunktionalen Reportermolekül; c) Einführen einer reaktiven Gruppe durch Umsetzen der terminalen funktionellen Gruppe mit einem bifunktionalen Linkermolekül. Vorteile dieser Ausführungsform ergeben sich aus der Möglichkeit, eine Markersubstanz wie hierin beschrieben erhalten zu können.

Ferner wird ein Verfahren zur Herstellung einer Markersubstanz und/oder eines modularen Baukastens vorgeschlagen, wobei das bifunktionale Reportermolekül, umfassend Chelatkomplex und reaktives Linkermolekül, ausgewählt ist unter: p-SCN-Benzyl-DOTA; p-Maleimid-Benzyl-DOTA; S-2-(4-Nitrobenzyl)-DOTA; S-2-(4-Aminobenzyl)-DOTA; DOTAsuccinimidylacetat; DOTA-N-hydroxysuccinimidester; S-2-(4-Aminobenzyl)-DTPA, p-SCN-Benzyl-DTPA; p-Maleimid-Benzyl-DTPA; Aminobenzyl-DO3A; p-SCN-Benzyl-oxo-DO3A; S-2-(4-Aminobenzyl)-NOTA; p-SCN-Benzyl-NOTA; p-SCN-Benzyl-Deferroxamin, Bromoacetamidobenzyl-DOTA (BAD); 2,2',2"-(10-(2,6-dioxotetrahydro-2H-pyran-3-yl)-1,4,7,10-tetraazacyclododecan-1,4,7-triyl)triacetic acid (DOTA-GA anhydrid). Vorteile ergeben sich aus den relativ milden Reaktionsbedingungen bei der Umsetzung dieser Linkermoleküle mit dem jeweiligen Trägermolekül. Der mit DOTA bezeichnete Chelatbildner weist eine besonders hohe Affinität für Lanthanoide auf.

Als Linkermoleküle für einen Linker, wie beispielsweise den ersten Linker, den zweiten Linker und/oder den dritten Linker, eignen sich Maleimid, Arylthiol, sulfoniertes Alkyl, Nitril, die alpha-Haloacylgruppe, ein Epoxid, Jodoacetylamid, die N-Hydroysuccinimidestergruppe, Isothiocyanat, Isocyanat, Sulfonylhalid, Tetrafluorphenylester, ein Säurehalid, ein Säureanhydrid, oder ein beliebiges carboxylsäurereaktives Amin.

Ferner wird ein Verfahren vorgeschlagen, wobei das bifunktionale Linkermolekül ausgewählt ist unter: m-Maleimidobenzoyl N-hydroxysuccinimidester (MBS); einem Carbodiimid; N,N'-Dicyclohexylcarbodiimid; 1-Ethyl-3(3-dimethylaminopropyl)-carbodiimid hydrochlorid (EDC); 2-Iminothiolan; Imidoester wie z.B. Dimethyl adipimidate (DMA); N-Succinimidyl-S-acetylthioacetat (SATA). Die Handhabung der benannten Linkermoleküle ist dem Praktiker vertraut, sie sind kommerziell verfügbar und bei geeigneten Lagerbedingungen auch hinlänglich stabil, so dass sie ohne weiteres auch als Komponenten eines Kits zur Markierung von Targets oder Zielen, wie beispielsweise Biomolekülen, bereitgestellt werden können. Daraus ergeben sich Vorteile für die Flexibilität in der Anwendbarkeit der die beschriebene Reportergruppe und/oder Markersubstanz nutzenden Analyseverfahren.

Ferner können Reportermoleküle ausgewählt sein unter organischen Quecksilberverbindungen wie p-hydroxymercuribenzoic acid (pHMB), Methylquecksilber (CH3HgCl) oder Ethylquecksilber (CH3CH2HGC1). Diese Reagenzien binden an Thiolgruppen.

Ferner können beim vorgeschlagenen Verfahren zur Herstellung einer Markersubstanz und/oder eines modularen Baukastens auf der Basis von Heteroelementmarkierten Biomolekülen die Reaktionsbedingungen zur Einführung einer auf das (Poly)peptid bezogen endständigen reaktive Gruppe so gewählt werden, dass die endständig eingeführte reaktive Gruppe nicht mit der Seitenkette einer im Trägermolekül vorhandenen Aminosäure reagiert. Insbesondere werden der pH-Wert, die Art und/oder Polarität eines Lösungsmittels, die Ionenstärke und/oder die Temperatur des Reaktionsansatzes so ausgewählt, dass die endständig eingeführte reaktive Gruppe nicht mit der Seitenkette einer im Trägermolekül vorhandenen Aminosäure reagiert. Das bietet den Vorteil, dass die Markersubstanz über die endständige Gruppe an den Container und/oder das Target gebunden werden kann. Daraus ergeben sich Möglichkeiten der Vereinheitlichung der Reaktionsbedingungen zur Umsetzung verschiedener Markersubstanzen mit dem Container und/oder Target.

Eine alternative Ausführungsform der Kopplung der Markersubstanz an den Container und/oder ein Target, insbesondere ein Biomolekül, ergibt sich aus der Verwendung eines geeigneten Enzyms, beispielsweise einer Transglutaminase, wie beschrieben von S. Jeger et al. (2010) in Angewandte Chemie 122:1-5.

Ferner wird ein Verfahren zur Herstellung einer wie hierin beschriebenen Markersubstanz und/oder modularen Baukastens vorgeschlagen, das weiterhin den Schritt umfasst: d) Maskieren einer Aminogruppe Gruppe durch Acetylierung. Die Acetylierung ist nur ein Beispiel, wie die Reaktionsfähigkeit einer funktionellen Gruppe zeitweise herabgesetzt, bzw. ausgeschaltet werden kann um mehrstufige Synthesen zu ermöglichen.

Ferner wird ein Nachweisreagenz vorgeschlagen, umfassend eine wie hierin beschriebene Markersubstanz und ein Molekül oder Fragmente eines Moleküls, das ausgewählt ist unter: einem Antikörper, umfassend IgG, IgM, und/oder IgE; Avidin; Biotin; einem Enzym; einem Enzymsubstrat; einem Hormon; Protein A; Protein G; einem Rezeptor; Streptavidin. Das Nachweisreagenz kann insbesondere als eine Füllung des Containers des modularen Baukastens vorgesehen sein.

Ferner wird ein Verfahren zur Quantifizierung und/oder zum Nachweis eines Biomoleküls und/oder Analyten in einer aus einem physiologischen Medium gewonnenen Probe vorgeschlagen. Das Verfahren kann optional einen Schritt der Probenaufbereitung umfassen. Während dieses Schritts wird der gesuchte Analyt angereichert und/oder von Begleitstoffen getrennt. Ebenso aber kann auf den Schritt der Anreicherung des Analyten verzichtet werden, wenn das mit der Markersubstanz, die über ein Target, insbesondere ein Biomolekül spezifisch an den Analyten gebunden wurde, erzeugte Signal hinlänglich groß, intensiv, hoch, deutlich bzw. das Signal/Rausch-Verhältnis einer spezifischen Messgröße ausreichend ist, um den Analyten in der jeweiligen Matrix qualitativ nachzuweisen und/oder zu quantifizieren. Insbesondere kann das Verfahren die Schritte umfassen: Kontaktieren eines Ziels mit dem modularen Baukasten, insbesondere mit einer Erkennergruppe des modularen Baukastens, Freisetzen von Markersubstanzen, die insbesondere als Füllung in dem Container vorgesehen sind.

Ferner kann das vorgeschlagene Verfahren zur Quantifizierung und/oder zum Nachweis eines Biomoleküls und/oder Analyten in einer Probe die Schritte aufweisen: Markieren eines Analyten mit einer Markersubstanz wie vorstehend beschrieben und Analysieren mittels Massenspektrometrie und/oder Atomspektroskopie. Dabei kann der Analyt ein charakteristisches Molekulargewicht aufweisen, das typischerweise bis zur Analyse mittels Massenspektroskopie und/oder Atomspektroskopie unverändert bleibt. Ebenso bleibt das quantitative Verhältnis der zur Analyse genutzten signalerzeugenden chemischen Elemente zueinander unverändert.

Der Schritt des Markierens kann beim vorgeschlagenen Verfahren zur Quantifizierung und/oder zum Nachweis eines Biomoleküls und/oder Analyten so erfolgen, dass ein den Analyten spezifisch bindendes oder vom Analyten spezifisch gebundenes Biomolekül, hier auch als Zielmolekül bezeichnet, mit der Markersubstanz modifiziert vorliegt und dieses Agens als Nachweisreagenz des Analyten anhand eines spezifischen Elementverhältnisses der Markersubstanz verwendet wird. Zum Feststellen des spezifischen Elementverhältnisses werden atomspektroskopische Methoden, beispielsweise Atomabsorptionsspektroskopie, Massenspektrometrie, Röntgenfluoreszenzanalyse oder andere elementspezifische Nachweisverfahren genutzt. Besonders bevorzugt werden dabei Nachweistechniken, die eine Unterscheidung zwischen verschiedenen Elementen der Lanthanoide / Lanthanide oder anderer der benannten Heteroelemente erlauben.

Das Verfahren kann den Schritt der Markierung des mit einer Markersubstanz nach markierten spezifischen Zielmoleküls umfassen. Ein derartiges Zielmolekül kann beispielsweise ein Antikörper, ein Antikörperfragment, eine antigenbindende Domäne eines Antikörpers, ein Enzym, ein Plasmid, ein Oligonukleotid oder eine Nukleinsäure sein.

Als Zielmoleküle kommen insbesondere Avidin, Streptavidin, Protein G und Protein A in Betracht. Die Zielmoleküle Protein A und Protein G binden spezifisch an bestimmte Antikörperklassen und können so genutzt werden, um Antikörper mit der Markersubstanz zu identifizieren. Ebenso können die Antikörper für ihren Einsatz im Immunoassay schonend mit der ProteinA/G-Markersubstanz modifiziert werden. In diesem Fall dient Protein A bzw. Protein G als bifunktionaler Linker.

Ähnliches gilt für die Anwendung von Avidin und Streptavidin, die eine spezifische Bindung mit Biotin eingehen. Wird die Markersubstanz an Avidin oder Streptavidin angebracht, können biotinylierte Zielmoleküle identifiziert werden bzw. im Falle von Antikörpern würden diese für den Einsatz im Immunoassay mit der Avidin-, bzw. Streptavidin-Markersubstanz modifziert werden. Beispiele für Biotinylierungsreagenzien sind Biotin-N-hydroxsuccinimidester und 3-(N-Maleimidopropionyl)biocytin.

Ferner kann die vorgeschlagene Markersubstanz genutzt werden, indem eine erste Markersubstanz ein erstes und ein zweites chemisches Element in einem bekannten Massenverhältnis zueinander aufweist und zur Modifizierung eines ersten Ziels, insbesondere eines ersten Biomoleküls, verwendet wird. So kann das erste Ziel, insbesondere das erste Biomolekül, an Hand des analytisch messbaren Massen-Verhältnisses der vorliegenden Elemente wiedererkannt werden. Ebenso können unbekannte Proben, die vorgeblich das erste Ziel, insbesondere das erste Biomolekül, enthalten, auf ihre Authentizität hin überprüft werden. Das ist insbesondere dann von Bedeutung, wenn ein spezifisches Bindungsvermögen des Ziels, insbesondere des Biomoleküls, zur Durchführung eines validierten Nachweisverfahrens genutzt werden soll, gleichzeitig aber die Verwendung anderer Ziele, insbesondere Biomoleküle, deren Spezifität nicht validiert wurde, von der ggf. unrechtmäßigen Verwendung in derartigen Nachweisverfahren ausgeschlossen werden sollen oder wenn deren Verwendung erkannt werden soll. Die Markersubstanz kann somit zur Authentifizierung von Zielen, insbesondere Biomolekülen, und/oder diese beinhaltenden Analyse-Kits oder von Bestandteilen solcher verwendet werden.

Ferner können die hier vorgeschlagenen Markersubstanzen und/oder modularen Baukästen zur Durchführung von Immunoassays verwendet werden. Vorteile ergeben sich gemäß spezieller Ausführungsformen, insbesondere wenn der Immunoassay ausgewählt ist unter: Enzym Immuno Assay (EIA), Enzym-linked Immunosorbent Assay (ELISA), Radioimmunoassay (RIA), Immunoblot, Western blot oder Southern blot, Microarrays. Die hohe Sensitivität und/oder universale Einsetzbarkeit zur Analyse der benannten Immunoassay-Formate bietet Vorteile hinsichtlich Zuverlässigkeit und Aussagekraft der erhobenen Befunde. Weiterhin können die Markersubstanzen in der Immunhistochemie Anwendung finden.

Ferner kann zumindest einer der vorgeschlagenen modularen Baukästen, Markersubstanzen oder Nachweisreagenzien verwendet werden, um die Präsenz, die Lokalisierung, und/oder die Konzentration einer Entität oder eines Bestandteils einer Entität zu bestimmen, die ausgewählt ist unter: einem Biopolymer, insbesondere einem Protein oder einer Nukleinsäure; einem Hormon; einem Rezeptor; einer Biomembran; einer Zelle; einem Mikroorganismus; einem Virus; einer bioaktiven Substanz; einem pharmazeutischen Wirkstoff. Vorteile ergeben sich beispielsweise für eine gesteigerte Nachweisempfindlichkeit und Zuverlässigkeit von Analyse- bzw. Untersuchungsergebnissen, beispielsweise in der Diagnostik, zur Therapieüberwachung oder in der Histochemie, sowie für die Rückverfolgbarkeit von Analyse- bzw. Untersuchungsergebnissen.

Ferner kann die vorgeschlagene Markersubstanz kovalent beispielsweise an einen Antikörper gebunden werden, insbesondere wenn die Markersubstanz als Füllung des Containers vorgesehen ist. Dabei kann der Antikörper ausgewählt sein unter Immunoglobulinen der Klassen IgG, IgM, und/oder IgE. Die vorgeschlagene Bindung der Markersubstanz an einen Antikörper erlaubt es, die Nachweisempfindlichkeit eines Analyseverfahrens für das jeweilige vom Antikörper erkannte Antigen weiter zu steigern (Signalamplifikation). Zusätzlich zu anderen, ggf. am Antikörper vorhandenen Markern, etwa Fluoreszenzfarbstoffen und/oder Enzymen und/oder Quantenpunkten kann der jeweils erhobene Befund mit Hilfe einer unabhängigen Nachweistechnik abgesichert werden. Aus der Verwendung unterschiedlicher Klassen von Antikörpern ergeben sich die mit diesen Antikörperklassen verbunden Vorteile. Insbesondere Antikörper der Klasse IgM zeichnen sich typischerweise durch eine hohe Avidität aus. Die Gewinnung von Antikörpern der Klasse IgM ist häufig dadurch erleichtert, dass die Etablierung von Zelllinien, die diese Antikörper in hoher Konzentration sezernieren erleichtert ist gegenüber jenen für Antikörper der Klasse IgG.

Ferner wird ein Verfahren zur Rückverfolgung und/oder zur verdeckten individuellen Kennzeichnung eines Targets, wie beispielsweise des Containers des modularen Baukastens, und/oder eines Ziels, wie beispielsweise eines Biomoleküls, vorgeschlagen. Das Verfahren umfasst die Schritte: A) Auswählen einer ersten Markersubstanz, wobei die erste Markersubstanz durch ein erstes Verhältnis der Mengenanteile von wenigstens zwei Heteroelementen zueinander gekennzeichnet ist; B) Kovalentes Binden der ersten Markersubstanz an das Biomolekül; C) in-Verkehr-Bringen des Biomoleküls, insbesondere durch Freisetzen des Biomoleküls aus dem Container des modularen Baukastens.

Ferner wird ein Verfahren zur Rückverfolgung und/oder zur verdeckten individuellen Kennzeichnung eines Biomoleküls vorgeschlagen, das weiterhin die folgenden Schritte umfasst: D) Durchführen einer atomspektroskopischen Analyse einer mutmaßlich das individuell gekennzeichnete Biomolekül beinhaltenden Probe; E) Ermitteln eines Verhältnisses der Massen- bzw. Mengenanteile von wenigstens zwei Heteroelementen zueinander in der mutmaßlich dass individuell gekennzeichnete Biomolekül beinhaltenden Probe; F) Feststellen der Identität oder der Nicht-Identität des festgestellten vom erwarteten Verhältnis der Massen bzw. Stoffmengen von wenigstens zwei Heteroelementen.

Ferner kann das bezeichnete Verfahren weiterhin den Verfahrensschritt umfassen: G) Zuordnen des Biomoleküls der Probe zu einer Liste bzw. das Feststellen der Zugehörigkeit oder das Nichtfeststellen der Zugehörigkeit zu einer Liste, bzw. das Feststellen der Nicht-Zugehörigkeit zu einer Liste. Vorteile dieses Verfahrensschritts ergeben sich aus der Validierbarkeit und Standardisierbarkeit von Analysen und Nachweisverfahren im analytisch/chemischen und diagnostisch/medizinischen Bereich, können sich aber ebenso im Bereich des Umweltmonitoring oder der Authentifizierung einer Produktidentität und der Fälschungssicherheit von Produkten und Erzeugnissen ergeben.

Die hierin beschriebenen Ausführungsformen können, auch nur teilweise, beliebig miteinander kombiniert werden.

Insgesamt wird somit ein Verfahren zur Markierung und/oder Identifizierung von modularen Baukästen und/oder Biomolekülen sowie für die Verwendung als Trägermaterial angepasste Aminosäuresequenzen, bzw. Peptide und Polypeptide, die zumindest teilweise mit einer Lanthanoid-haltigen Reportergruppe versehen sind, in diesem Verfahren vorgeschlagen.

Unter einem Peptid wird dabei die Verknüpfung von mindestens zwei Aminosäuren verstanden, wobei eine Aminogruppe einer ersten Aminosäure mit einer Carboxylgruppe einer zweiten Aminosäure kondensiert vorliegt. Damit ist das einfachste Peptid ein Dipeptid, das aus zwei miteinander kovalent verknüpften Aminosäureresten besteht. Ein Tetrapeptid weist dementsprechend drei Peptidbindungen auf und umfasst vier Aminosäuremoleküle. Ein Tetrapeptid kann als lineares Molekül mit mindestens zwei, jeweils endständigen funktionellen Gruppen vorliegen. Ein zyklisches Tetrapeptid weist hingegen keine endständige funktionelle Gruppe mehr auf, kann jedoch in Abhängigkeit von der Art der vier Aminosäure-Moleküle bzw. den in der Seitenkette vorhandenen funktionellen Gruppen, verschiedene funktionelle, d.h. reaktionsfähige Gruppen tragen.

Der vorgeschlagene molekulare Anhänger bzw. Marker ("molecular tag") besteht insbesondere aus einer Trägersubstanz und an die Trägersubstanz gebundener signalerzeugender Reportergruppen. Als Trägersubstanz werden Peptide vorgeschlagen. Ein für die Anwendung angepasstes Peptid umfasst eine Sequenz von derartigen Aminosäuren, die leicht mit den jeweiligen signalerzeugenden Reportergruppen (S1, S2, ..., Sx) markiert werden können.

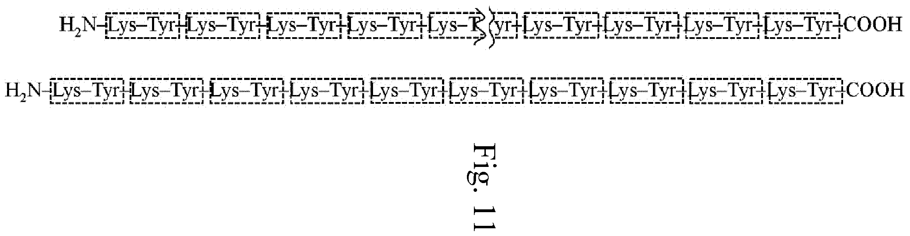

Ferner kann das als Trägermolekül einer Markersubstanz vorgeschlagene Molekül eine Vielzahl von miteinander über Peptidbindungen verketteten Peptid-Einheiten aufweisen, deren Aminosäuresequenz identisch ist. Beispielsweise kann ein solches Trägermolekül die peptidische Grundstruktur "RKYCS" aufweisen, wobei R, K, Y, C und S hier jeweils Aminosäuren, und zwar R - Arginin, K - Lysin, Y - Tyrosin, C - Cystein und S - Serin sind, die miteinander zu einem linearen Peptid der Sequenz RKYCS verbunden sind. Im entsprechenden "Polypeptid" sind mehrere solcher Grundstrukturen miteinander peptidisch verbunden. Das resultierende Trägermolekül kann beispielsweise die Struktur RKYCS-RKYCS-RKYCS-RKYCS-RKYCS haben. Die Peptidbindung zwischen benachbarten Aminosäuren innerhalb des Pentapeptids RKYCS ist identisch mit einer vorstehend als Strich "-" dargestellten Peptdidbindung zwischen Aminosäuren benachbarter Pentapeptide. Eine andere Schreibweise dieser Struktur ist (RKYCS)5. Um die frei bleibenden endständigen funktionellen Gruppen, beispielsweise für den Fall der hier vorliegenden 2-Aminocarbonsäuren (α-Aminosäuren) zu verdeutlichen, wird vereinfachend eine weitere Schreibweise gewählt, die diese funktionellen Gruppen zeigt: H2N-(RKYCS)5-COOH. Diese Art der Schreibweise ist in den Fig. 11 bis Fig. 14 verwendet, auch wenn dabei die Aminosäuren nicht mit einem Ein-Buchstaben-Code, sondern mit einem Drei-Buchstaben-Code dargestellt sind.

Die Zahl der ein Trägermolekül (Peptid) ausbildenden Aminosäuren kann ausgewählt unter 2 bis 150 sein, beispielsweise unter 3 bis 100, insbesondere unter 3 bis 50 und beträgt typischerweise zwischen 4 und 36.

Da Peptide automatisch synthetisiert werden können, zeichnet sich das routinemäßig erhaltbare Trägermaterial durch eine extrem gut-definierbare Heterogenität aus.

Als das mit der Markersubstanz und/oder der Reportgruppe markierte Target kommt der Container des modularen Baukastens in Betracht. Als die mit der Markersubstanz und/oder der Reportgruppe markierten Zielekommen Zielstrukturen und/oder Zielmoleküle, insbesondere Biomoleküle, wie beispielsweise Proteine, insbesondere Antikörper, Metaboliten oder Nukleinsäuren in Betracht. Diese Targets und/oder Ziele sind in der Lage, an unterschiedliche Strukturen (Entitäten) spezifisch zu binden (diese "zu erkennen") oder von diesen "erkannt" bzw. gebunden zu werden. Derartige Entitäten können beispielsweise in Lösungen, in physiologischen Flüssigkeiten, auf Zelloberflächen, an Membranen, auf Gewebeschnitten, auf oder in Mikroorganismen, im Inneren von lebenden oder fixierten toten Zellen und/oder auf dem Container des modularen Baukastens vorliegen. Ebenso aber können derartige Entitäten auf Feststoff- Oberflächen vorliegen.

Zum Nachweis der signalerzeugenden Reportergruppen können unterschiedliche Methoden eingesetzt werden. Insbesondere wird die Verwendung von: • optischen und photometrischen Methoden für den indirekten Nachweis einer oder mehrerer Reportergruppen, die eine Absorption, eine Fluoreszenz, Lumineszenz, Chemilumineszenz, eine Färbung bzw. einen Farbumschlag zur Signalgebung erzeugen kann; und von • atomspektrometrischen Methoden (Atomemissionsspektrometrie oder Massenspektrometrie) für den direkten Nachweis von signalerzeugenden Reportergruppen, die Heteroelemente enthalten, vorgeschlagen.

Dabei werden Heteroelemente ausgewählt, die im zu untersuchenden biologischen System selbst nicht vorkommen, wie z.B. seltene Metalle. Besonders geeignet sind hierzu die Lanthanoide. Dabei wird davon ausgegangen, dass das bei Detektion jeweils erzeugte Signal proportional zur Anzahl der eingeführten Reportergruppen ist. Insbesondere entspricht eine Intensität, eine Höhe oder eine Größe eines Messsignals der Anzahl der in der markierten Probe vorhandenen Reportergruppen. Damit ist bei durchgehend gleichmäßiger Markierung des nachzuweisenden Ziels das jeweils erhaltene Messsignal proportional zur vorliegenden Menge bzw. Konzentration des Ziels.

Eine Signalverstärkung wird erreicht durch Verwendung mehrerer, mittels gleicher Reportergruppen markierter, Aminosäuren. Das Vorliegen einer bestimmten Mischung verschiedener Reportergruppen kann zur Erzeugung eines Codes, bzw. zur Kodierung einer mit dem "molecular tag" markierten Entität genutzt werden. Eine solche Entität kann ein Protein, beispielsweise ein Antikörper; eine Nukleinsäure (DNA, RNA); eine Organelle, beispielsweise ein Zellkern; eine Zelle oder eine Zellgruppe; oder ein Mikroorganismus sein. Die beschriebene Kodierung bietet prinzipiell die gleichen Möglichkeiten, die in anderen Anwendungsfeldern ein Barcode bietet.

Das genutzte Trägermaterial kann entweder synthetisch erzeugt oder aus natürlichen Quellen (Polypeptide, Proteine) gewonnen werden und besteht aus miteinander über Peptidbindungen verknüpften Aminosäuren (X, Y,Z). Am Trägermaterial werden Heteroelemente gebunden. Einerseits können beispielsweise Halogene, wie z.B. Jod oder Brom direkt und kovalent an Aminosäuren gebunden werden (Jodierung, Bromierung etc.).

Ferner kann die Komplexbildung bestimmter Aminosäuren, bzw. Aminosäuresequenzen mit Heteroelementen, z.B. die Komplexbildung von poly-His mit Metall-Ionen zum Nachweis genutzt werden. Ferner können über extra eingeführte Reportergruppen Heteroelemente am Trägermaterial gebunden werden. Das eröffnet vielfältige Möglichkeiten der Einführung von Heteroelementen in das Trägermolekül.

Eine hohe Anzahl mit identischen signalerzeugenden Atomen oder Ionen markierter Aminosäuren eines Trägermoleküls (z.B. eines Oligopeptid) bewirkt im jeweiligen Detektorsystem ein höheres Detektorsignal, als ein kürzerer "molecular tag" und ermöglicht damit eine Steigerung der Empfindlichkeit der Nachweismethode bei der Untersuchung einer entsprechend markierten Probe. Die erreichbare Signalempfindlichkeit ist proportional zur Zahl der Markierungen in der genutzten Sequenz, wenn jeweils nur ein "molecular tag" am nachzuweisenden Biomolekül gebunden vorliegt.

Ein synthetisch erzeugtes Trägermaterial weist eine durch die gewählten Synthesebedingungen definierte Kettenlänge auf. Das ist von Vorteil für die exakte Quantifizierung einer mit dem entsprechenden "molecular tag" markierten Probe.

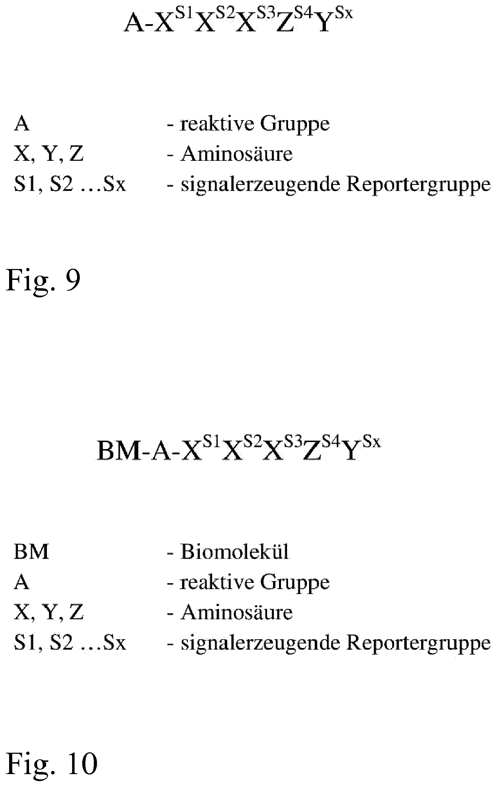

Der prinzipielle Aufbau und beispielhafte Ausführungsformen der entsprechenden "molecular tags" sind in den Fig. 9 bis Fig. 16 schematisch dargestellt.

Ausgestaltungen der Erfindung sind Gegenstand der Unteransprüche. Ausführungsbeispiele sind nachstehend unter Bezugnahme auf die beigefügten Zeichnungen näher erläutert.
Fig. 1 zeigt schematisch den Schritt der Elementmarkierung von identischen Biomolekülen.
Fig. 2 zeigt schematisch den Schritt der Elementmarkierung von Biomolekülen eines Biomolekülgemisches.
Fig. 3 zeigt schematisch den Schritt der Bindung der markierten Biomoleküle aus Fig. 1 an zugehörige biomolekülerkennende Spezies, die auf einer Oberfläche immobilisiert sind.
Fig. 4 zeigt schematisch den Schritt der Bindung der markierten Biomoleküle des Biomolekülgemisches aus Fig. 2 an die jeweils zugehörigen biomolekülerkennenden Spezies, die auf einer Oberfläche immobilisiert sind.
Fig. 5 zeigt schematisch den Schritt der Bindung von markierten molekülerkennenden Molekülen eines Typs an die zugehörigen Modifikationen der entsprechenden Biomoleküle.
Fig. 6 zeigt schematisch den Schritt der Bindung von unterschiedlich markierten molekülerkennenden Molekülen verschiedenen Typs an die zugehörigen Modifikationen der entsprechenden Biomoleküle.
Fig. 7 zeigt in einer schematischen Gegenüberstellung zwei Beispiele für ein Protein, das Träger verschiedener posttranslationaler Modifikationen ist, und die zugehörigen nach dem beschriebenen Verfahren vorgenommenen Markierungen.
Fig. 8 zeigt ein Übersichtsschema, das die Wechselwirkungen zwischen Benzol, Benzolmetaboliten sowie reaktiven Sauerstoffspezies (ROS) mit Signaltransduktionswegen und Enzymen, die zu benzolinduzierten Modifikationen regulatorischer Proteine in Knochenmarkszellen führen, illustriert.
Fig. 9 zeigt den allgemeinen Aufbau der vorgeschlagenen Peptidmarker.
Fig. 10 zeigt einen an ein Biomolekül gebundenen Peptidmarker.
Fig. 11 zeigt den prinzipiellen Bauplan eines Trägermoleküls am Beispiel von poly(Lysyl-Tyrosin) und deca(Lysyl-Tyrosin).
Fig. 12 zeigt ein Cysteinyl-Tyrosin - Trägermolekül.
Fig. 13 zeigt Trägermolekül das aus vier miteinander über Peptidbindungen verknüpften Lysyltyrosinylcysteinylserin-Peptiden aufgebaut ist (Polypeptid).
Fig. 14 zeigt ein Trägermolekül am Beispiel eines poly-Lysins.
Fig. 15 zeigt auf zweierlei schematische Art die Verknüpfung der Aminosäuren eines tetrapeptidischen Trägermoleküls.
Fig. 16 zeigt schematisch einige prinzipielle Möglichkeiten der Bindung von Heteroelementen am Trägermolekül und damit den prinzipiellen Aufbau der vorgeschlagenen Peptidmarker.
Fig. 17 zeigt schematisch Module eines erfindungsgemäßen Baukastens.

Die Figuren veranschaulichen Ausführungsformen und dienen zusammen mit der Beschreibung der Erläuterung der Prinzipien der vorgeschlagenen Vorrichtungen und Verfahren, insbesondere der molekularen Marker. Die Elemente der Zeichnungen sind relativ zueinander und nicht notwendigerweise maßstabsgetreu. Gleiche Bezugszeichen bezeichnen entsprechend ähnliche Teile.

Die vorliegende Erfindung beschreibt ein Baukasten-System, das aus einem oder mehreren einzelnen Modulen, also Bausteinen, aufgebaut und kombiniert werden kann, um das Drug-Discovery zu optimieren und zu steuern, insbesondere in Richtung Multiplexfähigkeit und/oder Hochdurchsatz-Bedingungen mit einfachem analytischen Nachweis.

Insbesondere beschreibt die vorliegende Erfindung ein Baukasten-System, das aus einem oder mehreren einzelnen Modulen, also Bausteinen, aufgebaut und kombiniert werden kann, um A) das Drug-Discovery zu verbessern und B) die lokale Anwendung von Wirkstoffen zu optimieren sowie C) Nebenwirkungen zu reduzieren. Vorzugsweise kann ein fertiges Objekt, das aus mehreren Modulen zusammengebaut worden sein kann, von außen, also außerhalb des Patienten, Kleintier, gesteuert und verfolgt werden.

Insbesondere im Falle A) kann der modulare Baukasten die Vorteile Multiplexfähigkeit und/oder Hochdurchsatz-Bedingungen, insbesondere mit einfachem analytischem Nachweis, aufweisen. Beispielsweise kann der Baukasten für das Drug-Discovery eingesetzt werden und kann anschließend gleich als Delivery System verwendet werden.

Die Vorteile des Baukastens können insbesondere im Falle B) darin liegen, dass nicht mehr der gesamte Organismus dem Wirkstoff ausgesetzt werden muss, sondern dass dieser nur lokal im erkrankten Gewebe/Organ zum Einsatz kommen kann. Dadurch lässt sich z.B. auch die Dosis erhöhen, die sonst den gesamten Organismus zu sehr belasten würde.

Der Vorteil der lokalen Anwendung kann insbesondere im Falle C) dazu genutzt werden, um in Therapien die Nebenwirkungen zu reduzieren oder um Substanzen applizieren zu können, die vom Körper abgebaut würden und so nicht zum Ziel gelangen könnten.

Vorzugsweise können alle Module zu jeder Zeit gleichzeitig, also simultan, mit spektroskopischen oder photometrischen Methoden nachgewiesen werden. Einzelne Elemente des Baukastens (beispielsweise Reportergruppen) können mit seltenen Erden markiert (gelabelt) werden, um ein analytisches Signal mit spektrometrischen Methoden zu erzeugen. Beispielsweise kann dadurch ein simulaten Messung von mehr als 40 Parameter ermöglicht werden Dieser Ansatz kann im Vergleich zu herkömmlichen Methoden, wie der immunhistologischen Färbung vorteilhaft sein, da bei den photometrischen Verfahren Elemente (Sekundärantikörper) verwendet werden, die eine Färbung von Geweben in der Mikroskopie hervorrufen, da sie gegen ungelabelte Erstantikörper gerichtet sind. Dadurch können diese herkömmlichen Methoden nicht im Multiplexbetrieb eingesetzt werden

Dieser Baukasten kann für die Qualitätskontrolle bei der Herstellung, für die Diagnostik zur Nachverfolgung der Transportwege sowie für die Verteilungen der Pharmaka (Wirkstoffe) im Organismus genutzt werden. So können Diagnostik und Freisetzung der Wirkstoffe, sogenannte Theranostik, kombiniert werden.

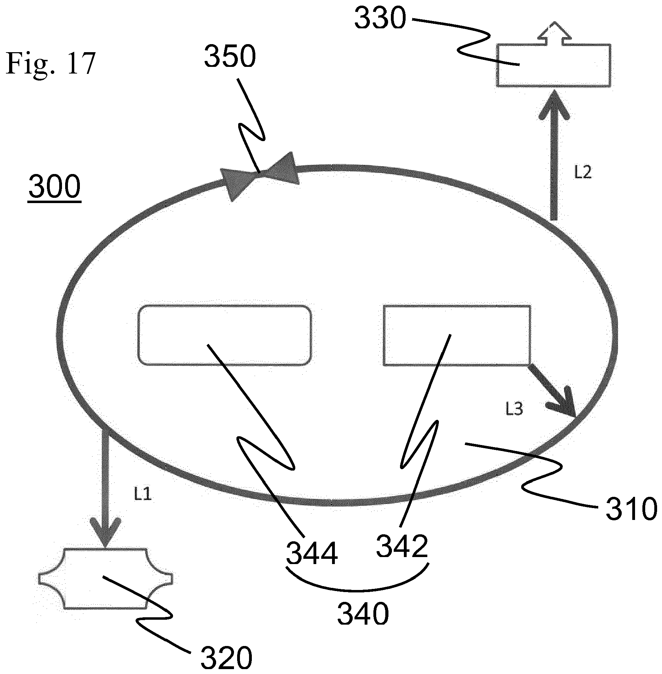

Die einzelnen Module des Baukastens 300 können allein oder in Kombination optimiert, getestet oder angewendet werden, wie in der Fig. 17 gezeigt.

Der Baukasten 300 kann maßgeschneiderte Anwendungen ermöglichen. Insbesondere kann für jede neue Anwendung auf bereits erprobte Module zurückgegriffen werden. Ein neuer Wirkstoff bedeutet nicht unbedingt eine Änderung der Module. Damit kann der Baukasten universell für die unterschiedlichsten Therapien und für unterschiedliche diagnostische Anwendungen eingesetzt werden.

Eine Mischung von Moduleigenschaften ist möglich und erweitert die Anzahl unterschiedlicher Anwendungen.

Ein Grundelement des Baukastens 300 besteht aus einem Container 310 (auch als Modul 0 bezeichnet), an das zur Detektion eine Reportergruppe, also ein Label, fest angebunden ist (auch als Modul 1 bezeichnet). Beispielhafte Reportergruppen sind hierin insbesondere mit Bezug auf die Fig. 9 bis 16 beschrieben. Ferner kann auch eine Erkennergruppe 330 angeschlossen werden (als Modul 3 bezeichnet).

In das Grundelement des Baukastens 300, also den Container, kann eine Füllung 340 (als Modul 4 bezeichnet) eingebracht sein. Die Füllung 340 kann beispielsweise eine Medikament, ein Wirkstoff und/oder ein Drug sein (auch als Modul 4.2 bzw. Modul 4.3 bezeichnet). Alternativ kann das Grundelement auch das Medikament und/oder der Wirkstoff und/oder das Drug selbst sein (auch als Modul 4.1. bezeichnet). In diesem alternativen Fall ist kein Container notwendig.

Die Füllung 340 im Container 310 kann mit einem Containermaterial fest verankert sein und kann bei Bedarf freigesetzt werden (als Modul 4.3 bezeichnet).

Die Füllung 340 im Container 310 kann frei und ungebunden vorliegen und kann nur nach dem Öffnen des Containers 310 direkt freigesetzt werden (als Modul 4.3 bezeichnet).

Im Nachfolgenden sind die einzelnen Module 1 bis 4 kurz skizziert.

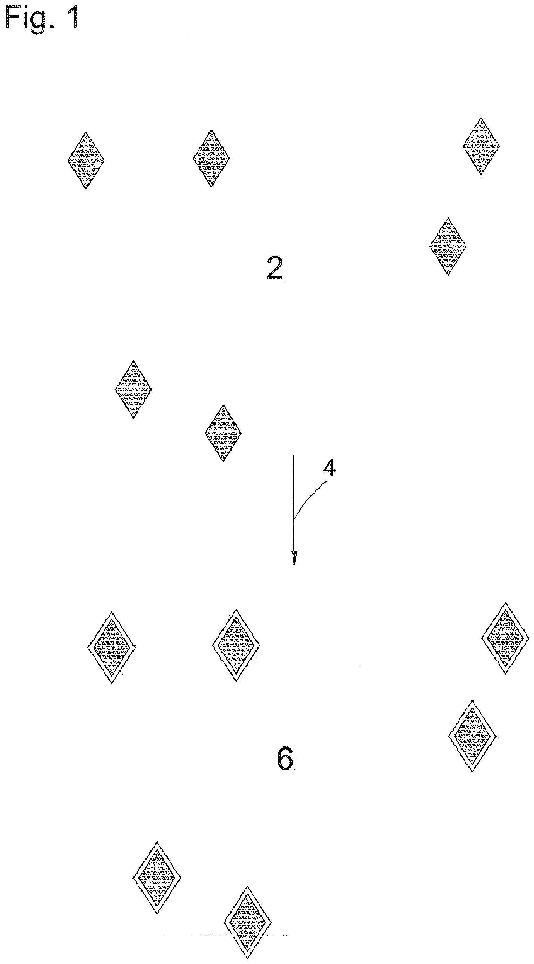

Besondere Eigenschaft der simultan-Detektion ist hierin insbesondere mit Bezug auf die Fig. 1 bis 8 beschrieben, deren Merkmale auf den erfindungsgemäßen Baukasten 300 übertragen werden können.

### Modul 1: Container

Der Container 310 kann einen Inhalt (Wirkstoff, Signal- oder Reportergruppe, Botenstoff, Inhibitor, etc.) aufnehmen, stabil lagern, transportieren schützen und/oder in einem Zielsystem passiv oder aktiv freisetzen.

Der Container kann ein Drug Delivery System, also ein Zuführsystem oder Freisetzungssystem eines Wirkstoffes, sein und/oder als Drug Delivery System verwendet werden.

Gemäß manchen Ausführungsformen kann in der Praxis auf bereits bekannte System zurückgegriffen werden. Es können jedoch auch eigens für den modularen Baukasten 300 konzipierte Container 310 verwendet werden.

Mit dem Container 310 kann mindestens einer Reportergruppe 320 verknüpft sein.

Der Container 310 wird von einer Wand umschlossen, die selbst Erkennereigenschaften haben kann.

Der Container 310 kann im geschlossenen Zustand eine Funktion ausüben oder einen Inhalt freisetzen. Das Material kann fest sein, so dass insbesondere keine Substanzen eindringen können. Das Material kann auch permeabel sein, um insbesondere bestimmte Substanzen hinein oder hinaus diffundieren zu lassen.

Der Container 310 kann von einer Wand umschlossen sein, die ein Schloss 350 aufweisen kann, mit dem der Container 310 geöffnet werden kann, um insbesondere seinen Inhalt freizugeben. Ferner kann der Container 310 auch nach Bedarf wieder verschlossen werden, insbesondere mit dem Schloss 350. Dadurch lässt sich die Freisetzung regulieren.

Der Container 310 kann einen Inhalt 340 oder Füllung 340 aufweisen, der entweder frei oder gebunden vorliegen kann.

Der Container 310 kann die Füllung 340 auf einmal oder dosiert freisetzen. In letzterem Fall kann so eine Depotwirkung über einen längeren Zeitraum erzielt werden.

Ein Container 310 kann mehrere, insbesondere verschiedene, Inhalte 340 oder Füllungen 340 aufweisen.

Die einzelnen Inhalte 340 oder Füllungen 340 können mit einer Reportergruppe 320 markiert sein.

Der Container 310 kann direkt mit mindestens einer Reportergruppe 320 verbunden sein.

Der Container 310 kann zusätzlich noch eine Erkennergruppe 330 enthalten. Die Erkennergruppe 330 kann beispielsweise dazu dienen, dass der Container an einer Zielstruktur andocken kann dort seine Füllung(en) 340 effektiv freisetzen kann, um dort einen Effekt zu erzielen. Beispielsweise wird in der konventionellen Chemotherapie ein Wirkstoff per Infusion angewendet und verteilt sich im gesamten Körper und kann so Organe schädigen (Nebenwirkungen). Durch die Erkennergruppe 340 kann der Container 310 an ein Ziel, wie eine erkrankte Zielstruktur, andocken und nur dort einen Wirkstoff in hoher Konzentration freisetzt, wodurch Nebenwirkungen reduziert und Dosierungen erhöht werden können.

Der Container 310 kann in der Größe und Oberflächenbeschaffenheit variiert werden, um sich den Eigenschaften eines biologischen System anzupassen, wie zum Beispiel ein Einsatz als ein sogenanntes "Trojanisches Pferd", und/oder um auf chemische und/oder physikalische Eigenschaften des Systems angepasst bzw. optimiert zu werden. Die Eigenschaften können beispielsweise Fließeigenschaften, hydrodynamische Eigenschaften, Dichte, Viskosität, Puffer, etc. sein.

Der Container 310 kann aus Nanomaterialien, wie beispielsweise Fullerenen, Buckminster-Fullerenen, Buckyballs, C60-Strukturen, Nanoröhrchen, Vesikeln, Dendrimeren, Liposomenn etc., oder biologischen Substanzen, wie beispielsweise Vesikeln, Liposomen, Zellen, Bakterien, Viren etc., gefertigt werden.

Der Container 310 kann auch ein einfaches, insbesondere offenes Molekül, wie beispielsweise ein Chelatbildner, ein Peptid, Protein, Antikörper, etc., sein oder ein, insbesondere abgeschlossenes, Objekt, wie beispielsweise ein Nanopartikel, ein C-Nano-Tube etc., sein, der in der Lage ist, seinen Inhalt zu schützen.

Der Container 310 kann das Schloss 350 aufweisen, dass insbesondere chemisch, beispielsweise über ein Enzym, pH-Wert und/oder Rezeptor, oder physikalisch, beispielweise über Wärme und/oder Strahlung, geöffnet oder gesteuert (öffnen-schließen, dosieren) werden kann.

### Modul 2: Reportergruppe 320

Die Reportergruppe 320 kann mit analytischen Methoden erkannt und detektiert werden.

Die Reportergruppe 320 kann über einen ersten Linker L1 mit dem Container 310 verbunden.

In einem modularen Baukasten 300 können mehrere Reportergruppen 320 verwendet werden, beispielsweise für einen Multiplexbetrieb.

Für die Unterscheidung der verschiedenen Reportergruppen 320 im Multiplexbetrieb sind massenspektrometrische oder fluoreszenzspektroskopische Verfahren besonders vorteilhaft. Es können auch andere atomspektrometrische Verfahren, wie beispielsweise optische Emissionsspektrometrie mit induktiv gekoppeltem Plasma (ICP-OES), Atomabsorptionsspektrometrie (AAS), etc., eingesetzt werden.

Die Reportergruppe 320 kann zur Lokalisierung und Verfolgung des Containers 310 dienen, ähnlich wie ein Barstrichcode beim modernen Paketdienst, um möglichst zu jeder Zeit abfragen zu können, wo sich der Container 310 in einem biologischen oder medizinischen Objekt befindet. So kann beispielsweise der ideale Zeitpunkt ermittelt werden, wann die Füllung 340 freigesetzt werden soll.

Beispielsweise kann die Reportergruppe 320 Atome, insbesondere Metalle zur Detektion in der Atomspektroskopie, wie beispielsweise Lanthanidlemente, Moleküle für oberflächenverstärkte Raman-Streuung und/oder organische Massenspektroskopie, Farbstoffe für die Mikroskopie, insbesondere Fluoreszenzfarbstoffe, aufweisen und/oder sein. Insbesondere können die Genannten als Label mit dem Container 310 verbunden, insbesondere kovalent verbunden, sein.

Die Reportergruppe 320 kann einer Verfolgung des Containers 310, einer Kodierung des Containers 310, und/oder einer Identifizierung des Containers 310, insbesondere wenn mehrere Container 310 eingesetzt werden, dienen.

Die Reportergruppe 320 kann Eigenschaften einer Erkennergruppe 330 enthalten.

Die Reportergruppe 320 kann sich insbesondere eignen, um Toxizitätsstudien durchzuführen, Kinetiken von Substanzen oder Wirkstoffen zu ermitteln, Verteilungen des Containers 310 oder seiner Inhaltsstoffe in Gefäßen, Geweben und/oder Organen insbesondere mit bildgebenden Techniken zu bestimmen, und/oder die Freisetzung des Inhaltes anzuzeigen.

Verschiedene Reportergruppen 320 können in einem Assay angewendet werden. Vorteilhaft ist es, wenn die verschiedenen Reportergruppen analytisch unterscheidbar sind. Insbesondere können die Reportgruppen 320 Multiplex- und Multiparameter Eigenschaften aufweisen und/oder für Multiplex- und Multiparameter Eigenschaften genutzt werden.

Der Einsatz von mehreren oder vielen Reportergruppen 320 in einem einzigen Experiment kann für ein High-Throughput-Screening genutzt werden.

Die Reportergruppe 320 kann analytisch nichtinvasiv, beispielsweise über Röntgen, MRI, Radioaktivität, im lebenden Objekt (Kleintier, Patient) nachweisbar sein, oder kann invasiv, beispielsweise als Gewebeprobe oder Biopsie, entnommen werden und kann dann mit spektroskopischen Methoden direkt im Gewebe oder im Gewebedünnschnitt messbar sein. Einige spektroskopische Methoden liefern ortsaufgelöste Signale, beispielswiese Röntgen-, Fluoreszenz- Raman-Mikroskop, LA-ICP-MS, und können so die Verteilung der Reportergruppen 320 in einem Ziel, wie einem Gewebe und/oder Organ, darstellen. So kann beispielsweise überprüft werden, dass der Wirkstoff tatsächlich an der richtigen Stelle freigesetzt werden kann.

### Modul 3: Erkennergruppe 330

Die Erkennergruppe 330 kann eine funktionelle chemische Verbindung, die ein Zielmolekül, wie beispielsweise ein Biomolekül, Zelle, Zellmembran, Peptide, Protein, DNA, RNA etc., und/oder eine Zielstruktur, wie beispielswiese ein Gewebe, Organ, Gefäß, etc., erkennen und/oder identifizieren kann und insbesondere über die Reportergruppe 320 des Containers 310 lokalisiert werden kann.

Die Erkennergruppe 330 kann über einen zweiten Linker L2 mit dem Container 310 verbunden sein.

Die Erkennergruppe 330 kann ferner über die Reportergruppe 320 des Containers 310 lokalisiert werden.

Die Erkennergruppe 330 kann selbst Reporteigenschaften aufweisen.

Die Erkennergruppe 330 kann ein chemisches Molekül sein, das insbesondere eine hohe Affinität zu einem Zielmolekül, wie beispielsweise ein Biomarker, ein Protein, Fett, Zucker, Ablagerung in einer Arterie, etc, oder einer Zielstruktur, wie beispielsweise ein Gewebe, Krebs, Organ etc., aufweist.

In einem einzigen Experiment können sich insbesondere viele Erkennergruppen 330 gleichzeitig testen lassen, wenn jede einzelne Erkennergruppe 330 mit einer unterscheidbaren Reportergruppe 320 ausgestattet sind. Dies kann beispielsweise zum Sreening unterschiedlicher Erkennergruppen 330 genutzt werden, um Substanzen mit hoher und/oder der höchsten Affinität für ein Ziel zu finden. Vorteilhafterweise können so neue in-Vivo-High-Throughput Möglichkeiten eröffnet werden.

Insbesondere kann die Erkennergruppe 330 ein Biomarker (Proteine) sein, die bei einer Krankheit in einem Gewebe (z.B. Krebs, Alzheimer, Parkinson) hochreguliert werden, und somit erkrankte Bereiche automatisch gefunden werden können.

Die Verwendung der Erkennergruppe 330 kann in der Praxis den Vorteil bieten, dass das Medikament (Drug) das Ziel, wie die Zielstruktur, selbst nicht erkennen muss. Das kann die Anzahl von Anwendungen erhöhen und die Drug-Entwicklung vereinfachen. Der modulare Baukasten 300 kann deshalb auf alle herkömmlichen Medikamente direkt anwendbar sein. Der modulare Baukasten 300 erfordert auch keine massgeschneiderten Medikamente. Der modulare Baukasten 300 kann sicher stellen, dass das Medikament an der richtigen Stelle bereitgestellt wird. Der modulare Baukasten 300 kann ferner den Einsatz von Medikamenten ermöglichen, die jetzt noch nicht eingesetzt werden dürfen, wegen zu hoher Nebenwirkungen oder zu geringer biologischer Stabilität.

Die Erkennergruppe 330 kann ein Antikörper, Biomolekül, Peptid, Polypetid, RNA, DNA, etc. sein.

### Modul 4: Füllung 340

Das Modul 4 beschreibt die Füllung 340 des Containers 310, insbesondere mit einer Substanz die in dem Ziel, wie beispielsweise der Zielstruktur, eine Wirkung hervorrufen kann. Die Füllung 340 kann beispielsweise ein Wirkstoff sein.

Gemäß einer alternativen Ausführungsform kann der die Füllung 340 ohne Container eingesetzt werden (siehe Modul 4.1).

Die Füllung 340 kann im Container 310 frei vorliegen (siehe Modul 4.2).

Die Füllung 340 des Containers 310 kann fest an oder im Container 310 verankert sein (siehe Modul 4.3).

### Modul 4.1.: Die Füllung 340 ohne Container 310

In diesem Fall kann die Füllung eine Substanz sein, wie beispielsweise ein Wirkstoff (Drug), ein Botenstoff, ein Inhibitor, ein Katalysator, eine radioaktive Substanz sein, die in der Lage ist, das Ziel, wie beispielsweise die Zielstruktur zu zerstören oder bildgebend abzubilden, ein Kontrastmittel für Röntgen oder MRI etc., eine Reportergruppe 320, ein Puffer, ein Stabilisator etc., die nicht durch einen Container 310 geschützt werden muss. An der Füllung 340 können direkt eine oder mehrere Erkennergruppen 330 und/oder Reportergruppen 320 angeheftet sein, insbesondere ohne die Funktion der Füllung 340 zu beeinträchtigen.

### Modul 4.3: Füllung 342 fest verankert

Die zu Modul 3 gehörende Füllung 342 kann mit dem Containermaterial über einen dritten Linker L3 verbunden sein und insbesondere in einem Biosystem solange verweilen bis der Container 310 geöffnet, ausgeschieden und/oder abgebaut wird.

Die Füllung 342 kann ein Wirkstoff, hierin auch als Drug bezeichnet, ein Botenstoff, ein Inhibitor, ein Katalysator, eine radioaktive Substanz, die insbesondere in der Lage ist, die Targetstruktur zu zerstören und/oder bildgebend abzubilden, ein Kontrastmittel für Röntgen und/oder MRI etc., und/oder eine Reportergruppe 320 etc. sein.

Bei Bedarf kann der dritte Linker L3 aus einer abbaubaren oder photolytischen Gruppe bestehen, die insbesondere chemisch, enzymatisch und/oder physikalisch geöffnet werden kann, um den Inhalt gezielt freizusetzen.

In einem Container 310 können mehrere Füllungen 342, 344 aus Modul 3 und 4 verwendet werden.

### Modul 4.2: Freie Füllung 344

Die zu Modul 4.2 gehörende Füllung 344 kann mit dem Containermaterial nicht gebunden sein. Ferner kann die zu Modul 4 gehörende Füllung 344 bei Bedarf direkt freigesetzt werden, insbesondere immer durch Diffusion oder Permeation (zum Beispiel bei Vorliegen einer permeablen Containerwand) und/oder zielgerichtet nach Öffnen des Containers 310.

Die Füllung 344 kann ein Wirkstoff (Drug), ein Botenstoff, ein Inhibitor oder ein Katalysator sein.

Die Füllung 344 kann weiterhin eine radioaktive Substanz, die beispielsweise in der Lage ist, die Targetstruktur zu zerstören und/oder bildgebend abzubilden, ein Kontrastmittel für Röntgen und/oder MRI etc., eine Reportergruppe 320 etc. sein.

### Ferner kann die Füllung ein Puffer und/oder ein Stabilisator etc. sein.

Bei Bedarf kann die Füllung 344 aus einer aktivierbaren Substanz bestehen, die beispielsweise chemisch, enzymatisch oder physikalisch aktiviert werden kann. Die Füllung 344 kann aus mehreren Komponenten bestehen, z.B. einem Puffer, um den Wirkstoff zu lösen, zu konservieren, im pH-Wert anzupassen oder bei Änderung des pH Wertes zu aktivieren.

Bei Bedarf kann die Füllung 344 aus einer abbaubaren oder photolytischen Gruppe bestehen, die beispielsweise chemisch, enzymatisch oder physikalisch geöffnet werden kann, um den Inhalt gezielt freizusetzen.

Die Füllung 344 kann aus mehreren Komponenten bestehen, z.B. einem Puffer, um den Wirkstoff zu lösen, zu konservieren, im pH-Wert anzupassen und/oder bei Änderung des pH Wertes zu aktivieren.

### In einem Container 310 können mehrere Füllungen 342, 344 verwendet werden.

Insbesondere können mit der vorliegen Erfindung simultane Multiplex-Experimente, beispielsweise für die Entwicklung neuer Kontrastmittel, zum Beispiel durch Einsatz von Lanthaniden/REE, durchgeführt werden. Durch Einsatz unterschiedlicher Lanthanide kann beispielsweise die Verteilung von Kontrastmitteln in Geweben und der Nachweis mittels Laser Ablation ICP-MS verbessert werden. Vorteilhafterweise kann die Verteilung in verschiedenen Organen an nur einem Tierexperiment durchgeführt werden. Dadurch können unterschiedliche reaktive Gruppen oder passive Seitenketten der Chelate, die für das MRI geeignet sind, erprobt werden. Die Verteilung kann simultan in Gewebe und Organen in nur einem einzigen Tierexperiment durchgeführt und gemessen werden. Nach der Messung ist insbesondere sofort belegbar, welche Änderung des Chelat-Grundgerüstes, welche Verteilung oder Anreicherung in Organen, Geschwüren, Krebszellen, Entzündungen etc. verursacht. Dies kann auch zur Exploration von Drug Discovery Systemen für Multiplex-Experimente genutzt werden.

Im Folgenden sollen anhand zweier Mausmodelle Aspekte der vorliegenden Erfindung erläutert werden. Die Mäuse können die folgenden Krankheitsmodelle aufweisen. Insbesondere können die Mäuse zur Erzeugung von pathologischen Proben verwendet werden.

### CLP-Modell: Mausmodell für Sepsis

Bei diesem Mausmodell gelangen intestinale Bakterien in die Peritonealhöhle und verursachen eine Entzündungsreaktion mit nachfolgender letaler Sepsis (CLP = cecal ligation and puncture). Zum Nachweis kann eine Entnahme von Nieren und ggf. der Leber erfolgen. Nach Entnahme kann eine Präparation der Gewebeschnitte erfolgen.

### LAD-Ligation: Mausmodell für Myokardinfarkt

Bei diesem Mausmodell erfolgt eine Induktion eines Myokardinfarkts mittels Ligation der linken Koronararterie (LAD = left anterior descending artery). Zum Nachweis kann eine Entnahme des Herzens und ggf. der Leber erfolgen. Nach Entnahme kann eine Präparation der Gewebeschnitte erfolgen.

Zur Überprüfbarkeit der Wirkungsweise der Erfindung kann eine Kontrollgruppe erstellt werden, von der ebenfalls die betreffenden Organe präpariert werden können.

Weiterhin sind hierin folgende Systeme, Gegenstände und Verfahren beschrieben, die ebenfalls Aspekte und Ausführungsformen der Erfindung betreffen können und/oder manche Ausführungsformen der Erfindung weiter spezifizieren können. So sollen Merkmale der folgende Systeme, Gegenstände und Verfahren, soweit sie nicht im Widerspruch mit dem soeben offenbarten stehen, mit dem soeben offenbarten kombiniert werden können, um weitere Ausführungsformen der Erfindung zu bilden.

Im Folgenden werden Ausführungsformen beschrieben, die als Ziel beispielhaft ein Biomolekül aufweisen. Die darin dargelegten Prinzipien können jedoch auch auf ein anderes Ziel, insbesondere ein Zielmolekül, das kein Biomolekül ist, und/oder eine Zielstruktur, übertragen werden, ohne von dem Offenbarungsgehalt abzuweichen. So kann anstelle des im Zusammenhang dieser Ausführungsformen beispielhaft beschriebenen Biomoleküls jedes andere geeignete Ziel verwendet werden.

In Fig. 1 ist eine Biomoleküllösung 2 gezeigt, allgemein eine Ziellösung, die nur eine Art von Biomolekülen, allgemein eine Art von Zielen, (angedeutet durch schwarze Rauten) enthält. Diese wird in einem Markierungsschritt 4 mit Element-Tags gleichen Typs versetzt. Diese Element-Tags gehen mit den Biomolekülen eine feste Verbindung ein und bilden so eine Lösung aus markierten Biomolekülen 6. Die Markierung wird durch die graue Umrandung der die Biomoleküle darstellenden Rauten angedeutet. Insbesondere kann die Erkennergruppe 330 und/oder die mindestens eine Füllung 340 des Containers 310 des erfindungsgemäßen modularen Baukastens 300 ein Element-Tag aufweisen.

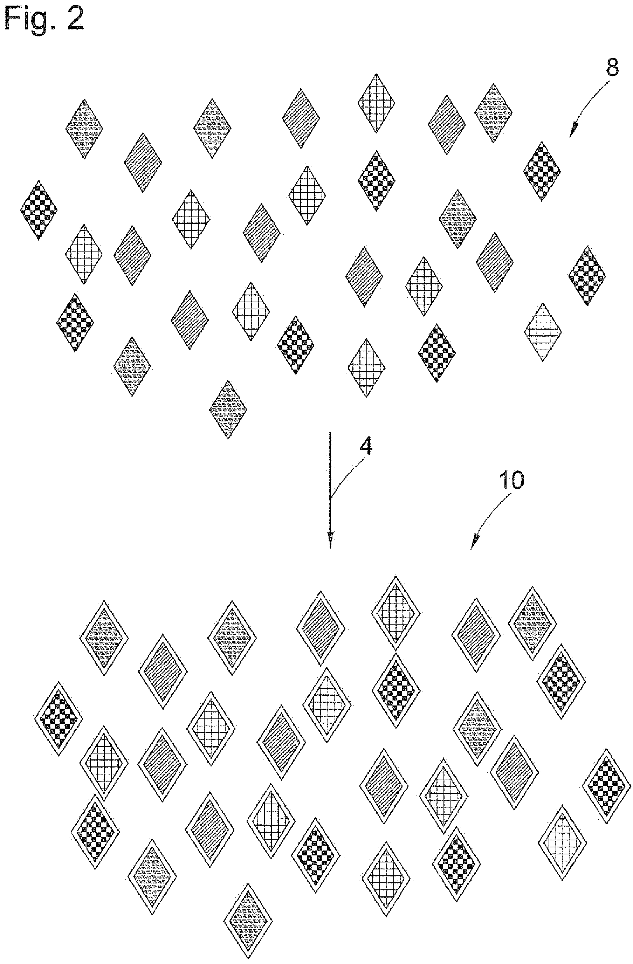

In Fig. 2 wird das System aus Fig. 1 dahingehend erweitert, dass mehrere Arten von Biomolekülen in einer Biomolekülmischung 8, allgemein Zielmischung, (angedeutet durch verschieden gemusterte Rauten) vorliegen. Diese werden ebenfalls in einem Markierungsschritt 4 mit Element-Tags gleichen Typs versetzt. Diese Element-Tags gehen mit den Biomolekülen eine feste Verbindung ein und bilden so eine Lösung aus einem Gemisch der so markierten Biomoleküle 10. Die Markierung wird durch die graue Umrandung der die Biomoleküle darstellenden Rauten angedeutet.

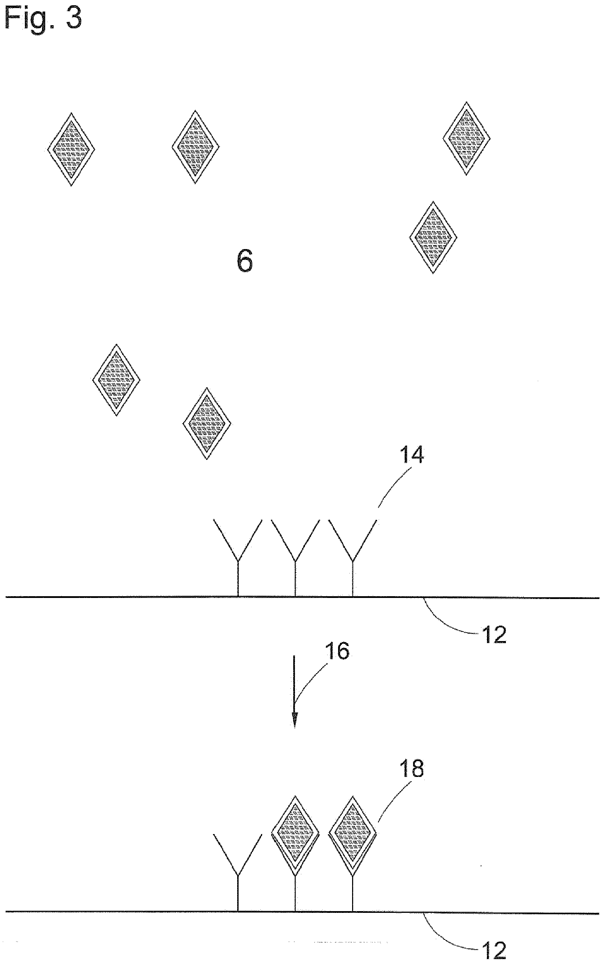

In Fig. 3 werden die markierten Biomoleküle 6 aus Fig. 1 in einem Schritt 16 mit auf einer Oberfläche 12 immobilisierten biomolekülerkennenden Spezies 14 kontaktiert. Die Oberfläche 12 kann insbesondere eine Oberfläche des Containers 310 des modularen Baukastens 300 sein. Beispielsweise kann die Erkennergruppe 330 die biomolekülerkennenden Spezies 14 aufweisen. Alternativ können die biomolekülerkennenden Spezies 14 auch als eine Füllung 340 des Containers 310 vorgesehen sein. In diesem Fall können die biomolekülerkennenden Spezies 14 nicht immobilisiert vorliegen. Die biomolekülerkennenden Spezies 14 sind spezifisch für die Biomoleküle aus der Biomoleküllösung 2 und binden diese. Es bildet sich ein Komplex 18 aus markierten Biomolekülen 6 und, gegebenenfalls auf der Oberfläche immobilisierten, biomolekülerkennenden Spezies 14.

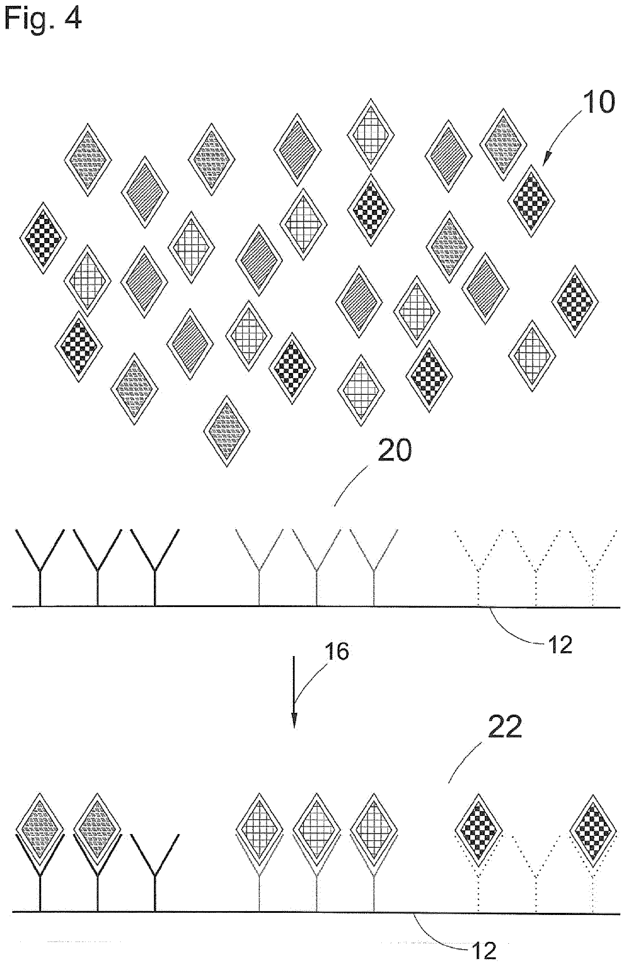

In Fig. 4 wird das markierte Biomolekülgemisch 10 aus Fig. 2 in einem Schritt 16 mit auf einer Oberfläche 12 immobilisierten biomolekülerkennenden Spezies 20 kontaktiert. Die Oberfläche 12 kann insbesondere eine Oberfläche des Containers 310 des modularen Baukastens 300 sein. Beispielsweise kann die Erkennergruppe 330 die biomolekülerkennenden Spezies 20 aufweisen. Alternativ können die biomolekülerkennenden Spezies 20 auch als eine Füllung 340 des Containers 310 vorgesehen sein. In diesem Fall können die biomolekülerkennenden Spezies 20 nicht immobilisiert vorliegen. Hier sind verschiedene Arten von biomolekülerkennenden Spezies vorgesehen bzw. auf der Oberfläche 12 verankert worden. Diese unterschiedlichen biomolekülerkennenden Spezies 20 sind jeweils spezifisch für bestimmte Biomoleküle aus der Biomolekülgemischlösung 8 und binden das jeweils zugehörige Biomolekül. Es bilden sich Komplexe 22 aus den verschiedenen markierten Biomolekülen 10 und den zugehörigen, gegebenenfalls auf der Oberfläche 12 immobilisierten, biomolekülerkennenden Spezies 22 verschiedenen Typs.

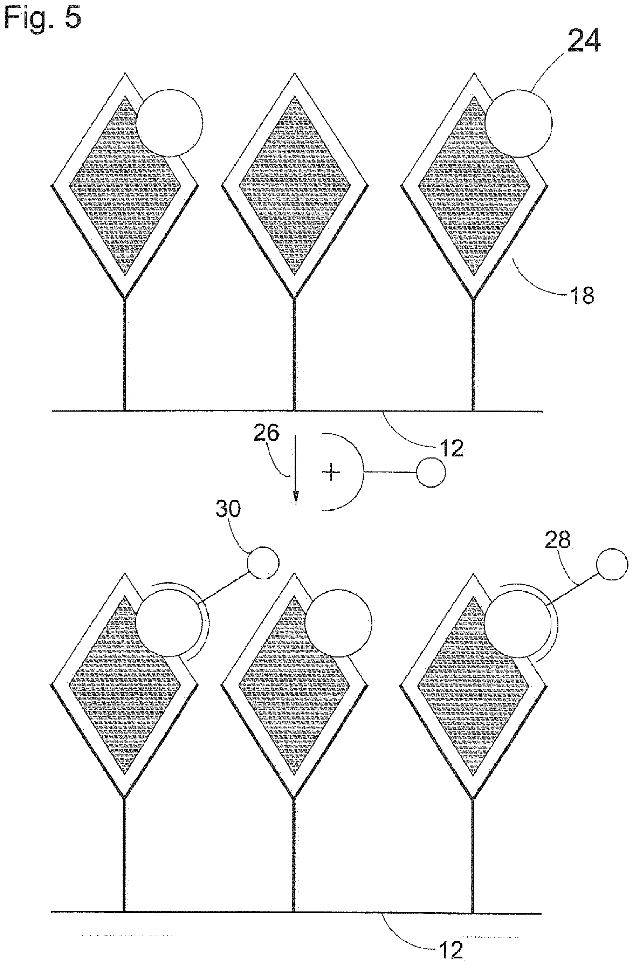

In Fig. 5 ist gezeigt, dass die entsprechend Fig. 3 in einem Komplex 18 an die, gegebenenfalls immobilisierten, biomolekülerkennenden Spezies gebundenen, markierten Biomoleküle Träger einer Modifikation 24 sind. Im Zusammenhang der vorliegenden Offenbarung kann die Modifikation 24 ebenfalls als Ziel verstanden werden. Insbesondere kann zwischen Biomolekülen und Modifikationen als zwei unterschiedliche Typen von Zielen unterschieden werden. In einem weiteren Markierungsschritt 26 werden diese Komplexe mit anders als das Biomolekül markierten modifikationserkennenden Molekülen, die aus dem modifikationserkennenden Molekül 28 und seiner Markierung 30 bestehen, kontaktiert. Insbesondere kann die Erkennergruppe 330 und/oder die mindestens eine Füllung 340 des Containers 310 des modularen Baukastens 300 das modifikationserkennende Molekül 28, vorzugsweise mit der Markierung 30, aufweisen. Das modifikationserkennende Molekül 28 bindet an die zugehörige Modifikation 24. Es bildet sich somit ein, gegebenenfalls auf der Oberfläche verankertes, System, das aus biomolekülerkennender Spezies, markiertem Biomolekül mit Modifikation und daran gebundenem markiertem modifikationserkennendem Molekül besteht. Mit einer geeigneten Detektionsmethode können nun die verschiedenen Markierungen identifiziert und quantifiziert werden. Damit werden indirekt das Biomolekül und seine Modifikation identifiziert und quantifiziert.

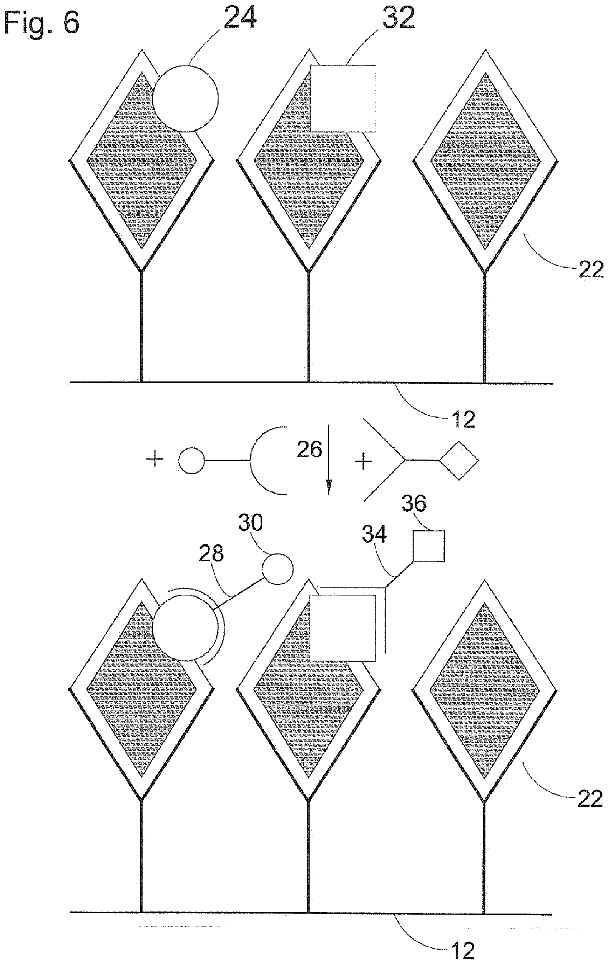

In Fig. 6 ist gezeigt, dass die entsprechend Fig. 4 in einem Komplex 22 an die immobilisierten biomolekülerkennenden Spezies gebundenen markierten Biomoleküle eines Typs sich untereinander in ihren Modifikationen 24, 32 unterscheiden. Hier wurde nur ein Biomolekültyp, allgemein Zieltyp, aus dem ursprünglichen Gemisch herausgegriffen, der exemplarisch auch für die anderen Biomoleküle der Mischung gilt. In einem weiteren Markierungsschritt 26 werden diese Komplexe mit anders als das Biomolekül markierten modifikationserkennenden Molekülen, die aus dem modifikationserkennenden Molekül 28 bzw. 34 und seiner Markierung 30 bzw. 36 bestehen, kontaktiert. Jedes modifikationserkennende Molekül bindet an die jeweils zugehörige Modifikation 24 bzw. 32. Es bildet sich somit ein, gegebenenfalls auf der Oberfläche 12 verankertes, System, das aus biomolekülerkennender Spezies, markiertem Biomolekül mit jeweiliger Modifikation und daran gebundenem entsprechendem markiertem modifikationserkennendem Molekül besteht. Mit einer geeigneten Detektionsmethode können nun die verschiedenen Markierungen identifiziert und quantifiziert werden. Damit werden indirekt das Biomolekül und seine unterschiedlichen Modifikationen identifiziert und quantifiziert. Selbstverständlich kann die Erkennergruppe 330 und/oder die mindestens eine Füllung 340 des Containers 310 des modularen Baukastens 300 mehrere, insbesondere unterschiedliche, modifikationserkennende Moleküle, wie die modifikationserkennenden Moleküle 28 und 34, vorzugsweise mit der jeweiligen Markierung 30 bzw. 36, aufweisen. Es ist sogar besonders bevorzugt, dass die mindestens eine Füllung 340 mehrere unterschiedliche modifikationserkennende Moleküle aufweist, um so beispielsweise ein Multiplex-Verfahren durchzuführen.

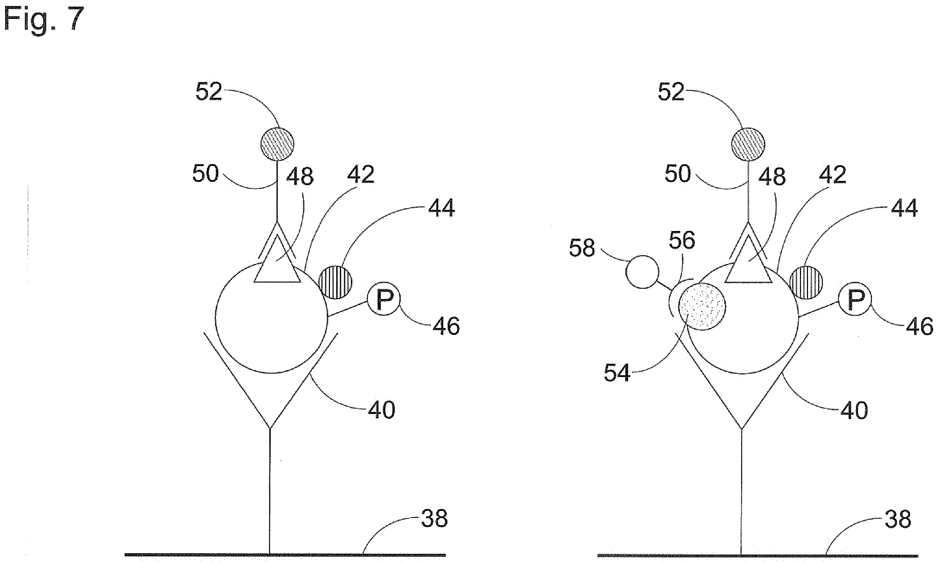

In einem konkreten Ausführungsbeispiel sind in Fig. 7 zwei mögliche Versionen von nach dem Verfahren und/oder mit dem modularen Baukasten 300 behandelten Biomolekülen dargestellt. Bei dem mit 42 bezeichneten Biomolekül handelt sich in beiden Fällen um das gleiche Protein p53. Im linken Fall ist das Protein 42 Träger einer Phosphatgruppe 46 aus einer posttranslationalen Phosphorylierung und Träger einer Zuckerfunktion 48 aus einer posttranslationalen Glykosylierung. Im rechten Fall trägt das Protein 42 zusätzlich eine SUMO-Gruppe 54. Das Protein 42 wird in beiden Fällen zunächst mit einem Eu-DOTA-Chelatkomplex 44 markiert. Auf einer Oberfläche 38, wie beispielsweise die Oberfläche des Containers 310 oder einer Gold-Mikroarrayoberfläche, wird die biomolekülerkennende Spezies 40 in Form des Antikörpers anti-p53 immobilisiert. Die Oberfläche wird mit der Lösung der markierten p53-Proteine in Kontakt gebracht, beispielsweise durch Freisetzen der Lösung aus dem Container 310 des modularen Baukastens 300. Die Antikörper anti-p53 gehen mit den europiummarkierten p53-Molekülen feste Bindungen ein. Dann werden modifikationserkennende Moleküle 50, 56 mit den Lanthanid-DOTA-Komplexen 52, 58 markiert. Für die Modifikation in Form einer Zuckergruppe 48 wird als modifikationserkennendes Molekül 50 ein mit einem Gd-DOTA-Chelatkomplex 52 markiertes geeignetes Lektin eingesetzt. Für die Modifikation in Form einer SUMO-Gruppe 54 wird als modifikationserkennendes Molekül 56 ein mit einem Sm-DOTA-Chelatkomplex 58 markierter Antikörper vom Typ anti-SUMO eingesetzt. Die modifikationserkennenden Moleküle 50, 56 werden mit den immobilisierten markierten p53-Proteinen kontaktiert. Die entsprechenden Modifikationen 48, 54 binden die zugehörigen modifikationserkennenden Moleküle 50, 56. Es bilden sich Systeme aus Protein p53 40 mit entsprechenden Modifikationen, Antikörper anti-p53 und an die Modifikationen gebundenen spezifischen modifikationserkennenden Spezies. Im linken Fall ist das Protein Träger von zwei Modifikationen, im rechten Fall Träger von drei Modifikationen. Das Verfahren lässt die Identifizierung und Quantifizierung von mehreren verschiedenen Modifikationen zu. Die Oberfläche 38wird in eine Laserablationszelle überführt. Das Proteinmaterial wird mit Hilfe eines Laserstrahls abgetragen und mit einem Transportgas in ein ICP-MS-Gerät überführt. Dort werden die verschiedenen zur Markierung eingesetzten Lanthanide der einzelnen Punkte der Oberfläche 38 vermessen. Gleichzeitig wird der in der Phosphatgruppe enthaltene Phosphor vermessen. Dies ist ein Beispiel für ein charakteristisches Strukturmerkmal einer Modifikation, die direkt bestimmt werden kann, ohne dass seine Markierung nötig ist. Der Antikörper 40 und die modifikationserkennenden Moleküle 50, 56 müssen dephosphoryliert vorliegen bzw. vor ihrem Einsatz gegebenenfalls mit Proteinphosphatasen dephosphoryliert werden, damit das Phosphorsignal in der Detektion eindeutig dem Protein 42 zugeordnet werden kann.

Die qualitative und quantitative Bestimmung der Elemente Eu, Gd, Sm und P mittels LA-ICP-MS gestattet in diesem Multiplexing-Ansatz die gleichzeitige Identifizierung und Quantifizierung des Proteins 42 und seiner jeweiligen Modifikationen 46, 48 und im rechten Fall der Fig. 7 auch 54.

Wie oben beschrieben, kann die Oberfläche 38 die Oberfläche des Containers 310 des modularen Baukastens 300 sein, wie es beispielsweise der Fall sein kann, wenn der modulare Baukasten 300 in einen Organismus eingebracht wird. In diesem Fall kann insbesondere die Erkennergruppe 330 die biomolekülerkennende Spezies 40 aufweisen. Das Protein 42 kann also auf einer Oberfläche des Containers 310 immobilisiert werden. Die mindestens eine Füllung 340 des Containers 310 kann die modifikationserkennenden Moleküle 50, 56, und insbesondere die Lanthanid-DOTA-Komplexe 52, 58, aufweisen. Nach Immobilisierung von Proteinen 42 auf der Oberfläche des Containers 310, können die modifikationserkennende Moleküle 50, 56, und insbesondere die Lanthanid-DOTA-Komplexe 52, 58, freigesetzt werden, um Systeme aus Protein p53 mit entsprechenden Modifikationen, Antikörper anti-p53 und an die Modifikationen gebundenen spezifischen modifikationserkennenden Spezies, die an den Container 310 des modularen Baukastens 300 gebunden sind, zu bilden. Das Freisetzen kann durch gezieltes Öffnen des Schlosses 350 erfolgen. Beispielswiese kann das Schloss 350 konfiguriert sein, um nach Immobilisierung von Zielen, wie das Protein 42, mit der Erkennergruppe 330 und/oder auf der Oberfläche des Containers 310 den Container 310 zu öffnen. Diese Systeme können den Organismus entnommen und entsprechend präpariert werden, um wie oben beschrieben vermessen zu werden.

Ferner kann die biomolekülerkennende Spezies 40 auch nicht auf einer Oberfläche mobilisiert sein. Beispielsweise kann der modulare Baukasten 300 in einen Organismus einbracht sein. Insbesondere dann kann die mindestens eine Füllung 340 des Containers 310 des modularen Baukastens 300 die biomolekülerkennende Spezies 40 und die modifikationserkennenden Moleküle 50, 56, und insbesondere die Lanthanid-DOTA-Komplexe 52, 58, aufweisen. Beispielsweise kann der modulare Baukasten 300 konfiguriert sein, um nach Einbringen in den Organismus, insbesondere nach einer bestimmten Zeit und/oder durch gezieltes Öffnen des Schlosses 350, die biomolekülerkennende Spezies 40 und die modifikationserkennenden Moleküle 50, 56, und insbesondere die Lanthanid-DOTA-Komplexe 52, 58, freizusetzen. In diesem Fall kann die biomolekülerkennende Spezies 40 auch über eine eigene Markierung (nicht gezeigt), wie beispielsweise die Lanthanid-DOTA-Komplexe, verfügen.

Wie oben beschrieben, kann die Oberfläche 38 die Oberfläche eines Mikroarrays sein. Ferner kann der Container 310 eine Erkennergruppe 330 in Form des Proteins 42 aufweisen. Die Erkennergruppe 330 kann also ein Molekül oder eine Struktur aufweisen, an die die biomolekülerkennende Spezies 40 bindet. Der modulare Baukasten 300 kann konfiguriert sein, um nach dem Binden bzw. Immobilisieren des modularen Baukastens 300, insbesondere der Erkennergruppe 330 des modularen Baukastens 300, an der biomolekülerkennende Spezies 40 bzw. auf dem Mikroarray die modifikationserkennende Moleküle 50, 56, und insbesondere die Lanthanid-DOTA-Komplexe 52, 58, freizusetzen.

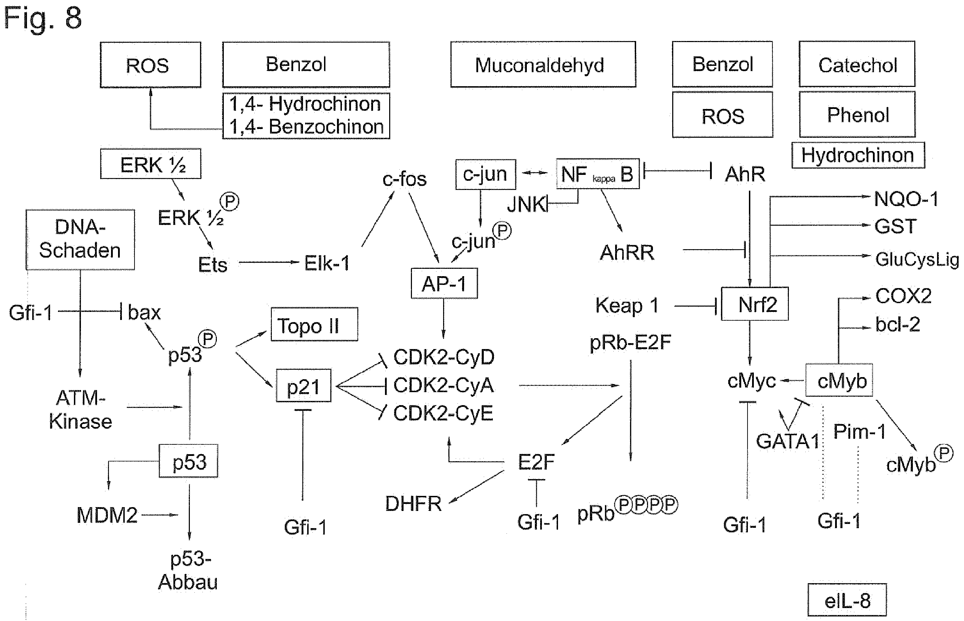

In Fig. 8 (die kleinen Kreise symbolisieren Phosphorylierungen, die den Aktivitätszustand der Proteine verändern) soll verdeutlicht werden, dass das Verfahren ein hohes Verwendungspotential in der Toxikologie, insbesondere in der Krebsforschung, hat. Benzol hat eine knochenmarktoxische Wirkung. Die Ursache dieser Wirkung ist die Bildung reaktiver Metaboliten, die kovalent an DNA, RNA und Proteine binden. Oxidationsprodukte des Benzols entstehen in der Leber und zum Teil auch im Knochenmark selber. Wichtige reaktive Metaboliten sind Benzolepoxid, Muconaldehyd, Phenol, Hydrochinon und Catechol. Der Eingriff dieser und anderer Metaboliten in Signaltransduktionswege und ihre Wechselwirkungen mit Enzymen führen zu Proteinmodifikationen von Proteinen, die an wichtigen Schaltstellen der Regulation von Zellteilung und Zelldifferenzierung stehen. Diese Proteine und ihre Modifikationen können mit dem Verfahren und/oder dem modularen Baukasten 300 simultan erfasst werden.

Neben der dargestellten Phosphorylierung von Proteinen kommen weitere Modifikationen wie zum Beispiel Ubiquitinylierung und Sumoylierung in Betracht. Der Nachweis dieser Modifikationen kann wie Zusammenhang der Fig. 7 beschrieben erfolgen.

Die in den Fig. 9 bis Fig. 16 gezeigte Grundstruktur der vorgeschlagenen Polypeptid-basierten Marker-Moleküle zur Markierung von Biomolekülen, nachfolgend verkürzt als "Polypeptidmarker" bezeichnet oder allgemein ein Markersubstanz, umfasst ein als Trägermolekül fungierendes Peptid, eine reaktionsfähige Gruppe und wenigstens eine signalerzeugende Reportergruppe, die an wenigstens einem der beteiligten Aminosäure-Reste gebunden ist. Gemäß hierin beschriebener Ausführungsformen kann der modulare Baukasten 300 die Markersubstanz einschließlich der Reportergruppe 320 aufweisen oder nur die Reportergruppe, wie sie im Zusammenhang mit den Marker-Molekülen beschrieben ist, alleine aufweisen.

Typischerweise weist das Trägermolekül miteinander über Peptidbindungen verknüpfte Untereinheiten auf. Diese Untereinheiten können vorteilhafterweise über Peptidbindungen miteinander verknüpfte Alpha-Aminosäuren sein. Weiterhin weist das Trägermolekül wenigstens eine endständige reaktive Gruppe auf. Unter einer Peptidbindung wird dabei verstanden, dass je eine Aminogruppe und eine Carboxylgruppe beteiligter Aminosäuren am gleichen Kohlenstoffatom gebunden sind und dass die Peptid-Bindung zwischen einzelnen Aminosäuren auf dem Wege der Kondensation derartiger Aminogruppen und Carboxylgruppen miteinander zu Stande kommt. Daraus ergibt sich, beispielsweise, das ein aus Lysin-Bausteinen aufgebautes Peptid im Sinne eines hier beschriebenen Trägermoleküls, also ein poly-Lysin, anders aufgebaut ist als ein, beispielsweise in der Zellkultur zur Oberflächenmodifizierung verwendetes, Polylysin, dessen Verknüpfungen Säureamide mit der Epsilon-Aminogruppe des Lysin darstellen.

Das so aufgebaute Di-, Tri-, Tetra-, ... Oligo-, bzw. Polypeptid weist, neben ggf. in Seitenketten der beteiligten Aminosäurereste vorhandenen funktionellen Gruppen, wenigstens zwei endständige reaktionsfähige Gruppen auf. Typischerweise sind das eine endständige Aminogruppe und eine endständige Carboxylgruppe. Beispiele für Aminosäuren, die als Bausteine für die hier beschriebenen Trägermoleküle in Frage kommen, sind, aufgeführt mit einem gebräuchlichen Kürzel und dem entsprechenden Trivialnamen: Ala (Alanin); Arg (Arginin); Asn (Asparagin); Asp (Asparaginsäure); Cys (Cystein); Glu (Glutaminsäure); Gln (Glutamin); Gly (Glycin); His (Histamin); HyLys (Hydroxylysin); HyPro (Hydroxyprolin); Ileu (Isoleucin); Leu (Leucin); Lys (Lysin); Met (Methionin); Phe (Phenylalanin); Pro (Prolin); Ser (Serin); Thr (Threonin); Try (Tryptophan); Tyr (Tyrosin); Val (Valin). Eine besondere Gruppe stellen Cystein, Methion, Selenocystein dar, da diese bereits ein Reporterelement enthalten (S, Se), welches zum Nachweis herangezogen werden kann.

Gemäß weiteren Ausführungsformen werden ausdrücklich nicht ausschließlich proteinogene Aminosäuren zum Aufbau der Trägersubstanz verwendet. Beispiele anderer, ebenso zum Aufbau geeigneter Trägermoleküle gleichwertig einsetzbarer Aminosäuren sind Ornithin, Citrullin, DOPA, Homoserin oder Thyroxin. Dem Fachmann sind weitere Aminosäuren bekannt. Dabei wird hier bewusst nicht unterschieden zwischen den D- und L-Formen der Aminosäuren, da für einige der hier vorgesehenen Anwendungen die Chiralität der Aminosäuren nicht zwingend der in der belebten Natur anzutreffenden entsprechen muss. Insbesondere, wenn der Einsatz des Markermoleküls zu analytischen Zwecken außerhalb des Körpers oder überhaupt unabhängig von jeglichen physiologischen Bedingungen erfolgt, können auch typischerweise nicht-proteinogene D-Aminosäuren verwendet werden.

Beispielsweise kann die beschriebene Markersubstanz einschließlich der Reportergruppe 320 und/oder die Reportergruppe 320 zur Verstärkung und/oder Erzeugung eines Signals dienen, das auf die selektive Bindung mit Hilfe eines Antikörpers gewonnen wird, der zum spezifischen Nachweis von Antibiotika, Pestiziden, Pestizidrückständen oder anderen Substanzen mittels angepasster Nachweistechniken (z.B. Enzymimmunoassay (EIA), ELISA, entsprechende Mikrospot-Arrays, partikelgestützte Nachweisverfahren) oder entsprechenden Sensorsystemen Verwendung findet.

Auch ist die Auswahl der verwendbaren Aminosäuren nicht auf α-Aminosäuren beschränkt. Beispielsweise kann als endständige Aminosäure des Trägermoleküls eine β-Aminosäure, beispielsweise β-Alanin, oder eine γ-Aminosäure, beispielsweise γ-Aminobuttersäure (GABA) oder auch eine Aminosulfonsäure eingesetzt werden.

In der Konsequenz der Verwendung einer dieser "Nicht- α-Aminosäuren" als terminale Aminosäure des vorgeschlagenen Markermoleküls, kann die Vielfalt der zur Verfügung stehenden "terminalen" funktionellen Gruppen erhöht werden.

Im Fall der Verwendung eines zyklischen Peptids als Trägermaterial wird die Funktion der terminalen funktionellen Gruppe durch eine entsprechend ausgewählte funktionelle Gruppe einer Seitenkette der zu einer Ringstruktur miteinander verbundenen Aminosäuren erfüllt. Diese funktionelle Gruppe kann während der Peptidsynthese geschützt sein und erst nach Ringschluss entschützt werden.

Die signalerzeugende Reportergruppe 320 umfasst typischerweise einen Chelat- oder Komplex-Bildner, der ein Heteroelement aufweist. Unter einem Heteroelement werden im vorliegenden Zusammenhang beliebige chemische Elemente verstanden, ausgenommen Wasserstoff, Kohlenstoff, Stickstoff und Sauerstoff. Vorteilhafterweise werden für die vorgeschlagene Anwendung derartige Heteroelemente gewählt, die in der jeweiligen Probenmatrix nicht oder in nicht störenden Mengen vorkommen. Als Probenmatrix werden in diesem Zusammenhang beispielsweise eine physiologische Flüssigkeit, ein Serum, eine Zelle, ein Zellverband, ein Zell-Extrakt, ein Zell-Aufschluss, ein Biopsat, ein Abstrich, ein Gewebe, ein Gewebsaufschluss, eine Zellkulturflüssigkeit, ein Gewebeschnitt, eine Chromatographie- oder eine Elektrophorese-Fraktion verstanden. Als Probenmatrix kann in diesem Zusammenhang auch die Umgebung des modularen Baukastens 300 verstanden werden, wenn der modulare Baukasten 300 in einen Organismus eingebracht wird. Ebenso ist es von Vorteil, wenn das Heteroelement nicht in den zur Handhabung der jeweiligen Probe verwendeten Hilfsmaterialien und Utensilien vorkommt. Derartige Hilfsmaterialien und Utensilien sind beispielsweise chromatographische Trägermaterialien oder zum Abklatsch (Blot) verwendete Papiere oder Folien, beispielsweise Nitrozellulose oder Nylon, sowie Laborgeräte und Einwegmaterialien, wie etwa Mikrotiterplatten, Pipettenspitzen oder Schutzhandschuhe. Ebenso ist das Heteroelement vorteilhafterweise nicht in Wasch-, Puffer- und Inkubationslösungen enthalten.

Typische Anwendungen der vorgeschlagenen Markersubstanz umfassen die Erzeugung und/oder die spezifische Verstärkung eines Signals, das auf die selektive Bindung eines Antikörpers an einem Molekül, einem Rezeptor, einer Oberflächenstruktur, einem Membranbestandteil, einem Mikroorganismus, einer Pilzhyphe, einem Virus, einer Organelle, oder einem Gewebe zurückgeht. Die Markersubstanz und/oder die Reportergruppe 320 kann somit vorteilhafterweise nicht nur zur Identifizierung des Containers 310 verwendet werden, die Markersubstanz und/oder die Reportergruppe 320 kann auch als Markierung, wie beispielsweise die Markierungen 30, 36, 44, 52, 58 für die hierin beschriebenen modifikationserkennenden Spezies, dienen. Dabei kann ein mit Hilfe der Heteroelement-Markierung erzeugtes spezifisches atomspektroskopisches Signal (z.B. eine Spektrallinie, eine Massenzahl, ein Energiespektrum) als alleiniges Signal zur Identifikation der Markersubstanz bzw. der Reportergruppe 320 und der mit ihr markierten Entität dienen. Ebenso aber kann eine andersartige Reportergruppe, beispielsweise ein Fluoreszenzfarbstoff oder ein Enzym, die an identischen Antikörpern oder an Antikörpern einer identischen Spezifität gebunden vorliegen, zur Multispektralanalyse einer Probe herangezogen werden. Beispielsweise kann ein Typ der hier vorgeschlagenen Markermoleküle gemeinsam mit einem anderen spezifischen Nachweisreagenz zur Bildgebung genutzt werden. Derartige Nachweisreagenzien können für diagnostische Fragestellungen, für pharmakokinetische Untersuchungen und/oder für die biomedizinische Forschung von Interesse sein. Auf Anwendungen im Bereich der Produktidentifikation wurde bereits hingewiesen.

Eine typische Struktur des mit Reportergruppen versehenen Trägermaterials ist in Fig. 9 gezeigt. Dabei stellt "A" eine funktionelle Gruppe, beispielsweise eine Aminogruppe dar. Dem Fachmann sind zahlreiche Methoden bekannt und entsprechende Reagenzien zugänglich, die unter milden und physiologischen Bedingungen die direkte oder indirekte kovalente Bindung einer Aminogruppe an ein Target-Molekül gestatten. Üblicherweise werden als "Linker" bezeichnete niedermolekulare Verbindungen eingesetzt, um ein Target, wie und/oder ein Targetmolekül, wie die modifikationserkennende Spezies, mit der beschriebenen Markersubstanz zu modifizieren.

Fig. 10 zeigt schematisch ein Biomolekül "BM", das über eine Gruppe "A" mit einer Sequenz von Aminosäuren X-X-X-Z-Y verbunden ist. Die Aminosäuren tragen jeweils spezifische signalerzeugende Reportergruppen S1, S2, S3, S4, Sx. Dabei ist die signalerzeugende Reportergruppe 320 S so ausgewählt, dass der Nachweis des markierten Biomoleküls Targets, wie beispielsweise des Containers 310 des modularen Baukastens 300 und/oder eines Biomoleküls, folgenden Anforderungen genügt: 1) qualitative Nachweisbarkeit; 2) quantitativ Nachweisbarkeit; 3) Unterscheidbarkeit, bzw. Möglichkeit der Codierung; 4) Signalverstärkung gegenüber herkömmlichen Markierungen.

Die Aminosäuresequenz kann einerseits eine Reihenfolge verschiedener Aminosäuren sein, kann aber auch eine Folge unterschiedlicher Gruppen von Aminosäuren (Peptide) umfassen. An einer derartigen Gruppe von Aminosäuren, beispielsweise an einem Tetrapeptid, können verschiedene Reportergruppen angekoppelt sein, so dass das markierte Trägermaterial, d.h. der Peptidmarker, verschiedene Reportergruppen S1...S4, bzw. im allgemeineren Fall S1...Sx in einem bestimmten quantitativen Verhältnis tragen kann.

Das feste quantitative Verhältnis der Reportergruppen bietet die Möglichkeit der Erzeugung jeweils charakteristischer Signal-Muster (Signatur) beim Nachweis der Heteroelemente. Das wiederum ermöglicht eine elementbasierte Kodierung der modifizierten Targets, wie beispielsweise des Containers 310 des modularen Baukastens 300 und/oder eines Biomoleküle.

Fig. 11 zeigt schematisch den Aufbau eines aus einander wiederholenden Lysyl-Tyrosin-Einheiten aufgebauten Trägermoleküls. Es handelt sich somit um ein Lysyltyrosin-Polypeptid. Wie ersichtlich, ist die jeweils gewählte Länge des Trägermoleküls frei wählbar und kann der gewünschten Anwendung angepasst werden. Die Seitenketten des Tyrosins können direkt mit Jod modifiziert werden. Ebenso kann Jod aber auch direkt an Histidin gebunden werden, wenn Histidin in einem entsprechenden Trägermaterial vorliegt

Die Seitenketten der Lysin-Moleküle können über die Bindung von p-SCN-Benzyl-DOTA mit verschiedenen Lanthanoiden befrachtet werden. Dazu kann die endständige Carboxylfunktion zur Kupplung des Chelatbildners an das Target-Molekül benutzt werden. Mit Bezug zu den Anwendungsgebieten der vorgeschlagenen Peptidmarker bietet z.B. die Jodierung über die Auswahl spezifischer Isotope die Vorteile der Anwendung der für diese Isotope bekannten bildgebenden Verfahren der biochemischen bzw. medizinischen Diagnostik. Beispielsweise kann neben <127>I auch <123>I oder <125>I verwendet werden.

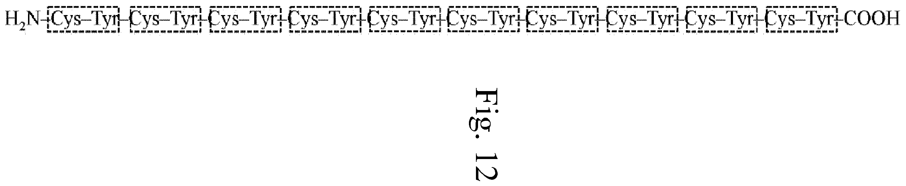

Fig. 12 zeigt eine weitere Ausführungsform eines Trägermoleküls, umfassend eine Cysteinyl-Tyrosin-Sequenz. Auch hier ist die Länge des Trägermoleküls einstellbar und wird praktischerweise an die jeweilige Anwendung angepasst. Tyrosin kann leicht halogeniert, beispielsweise problemlos jodiert werden. Ein weiteres Heteroelement kann über die Bindung je eines p-Maleimid-Benzyl-DOTA pro Cystein-Seitenkette eingeführt werden. Die endständige Carboxyl- und Aminofunktion können zur Kupplung an das Target benutzt werden.

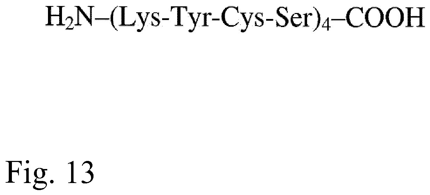

Fig. 13 zeigt ein aus vier identischen Blöcken aufgebautes Trägermolekül. Dieses Trägermolekül hat ein Molekulargewicht von MW = 2404,69 g/Mol. Es hat je 4 Kupplungsstellen folgender Art: -NH2; -C6H4OH; -SH und -OH. Die endständige Carboxylfunktion kann zur Kupplung benutzt werden.

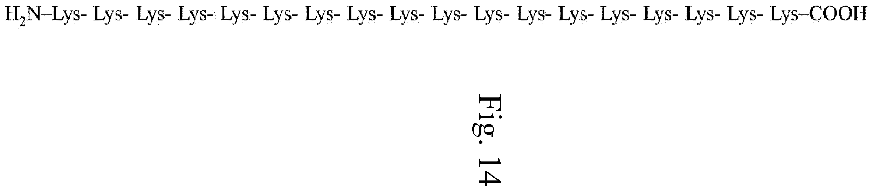

Fig. 14 zeigt ein aus 18 Lysinmolekülen aufgebautes lineares Peptid. Besondere Vorteile bieten die einer selektiven Modifizierung mit den vorstehend erwähnten bifunktionellen Reportermolekülen zugänglichen ε-Amino-Gruppen der Seitenketten des Lysins. Sie erlauben eine besonders hohe Beladung mit signalerzeugenden Heteroelementen, beispielsweise mit Lanthaniden. Das gezeigte Molekül unterscheidet sich deutlich von dem in der Zellkultur verwendeten Polylysin, dessen Verknüpfungen Säureamide mit der ε-Aminogruppe des Lysins darstellen. Das hier zu verwendende Molekül enthält tatsächliche über die α-Aminogruppe geknüpfte Peptidbindungen. Markierungen können z.B. mit Isothiocyanato-Verbindungen der chelatbildenden Gruppen erzielt werden, insbesondere mit 2- (4-Isothiocyanatobenzyl)-1,4,7,10-tetraazacyclododecan-1,4,7,10-tetraessigsäure.

Die positiven Ladungen von aus sterischen Gründen nicht modifizierten ε-Aminogruppen können gegebenenfalls durch Umsetzung mit kleinen NH2-reaktiven Molekülen neutralisiert werden. Die endständige Carboxylfunktion kann zur Kupplung benutzt werden.

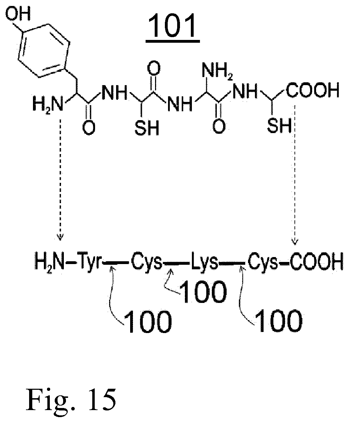

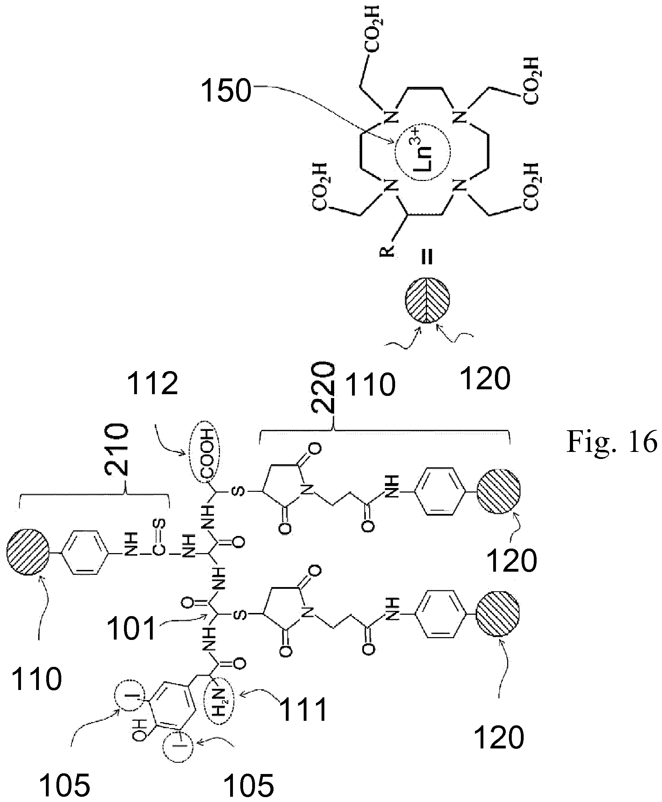

Die Fig. 15 und Fig. 16 veranschaulichen zusammenfassend typische Merkmale und Besonderheiten des hier vorgeschlagenen Peptidmarkers. Dabei zeigt Fig. 15 ein Trägermolekül 101 in Gestalt eines Tetrapeptids Tyr-Cys-Lys-Cys, bzw. YCKC. Die Aminosäurebausteine sind über Peptidbindungen 100 miteinander verknüpft. Im Formelschema ist die chemische Struktur der Aminosäuren vereinfacht dargestellt, insbesondere ist die Seitenkette des Lysin nicht gezeigt, sondern nur die ε-Aminogruppe.

Fig. 16 zeigt am Beispiel dieses Trägermoleküls 101 die endständige Aminogruppe 111 sowie die endständige Carboxylgruppe 12, die beide zur Kopplung des Peptidmarkers an das Target (hier nicht gezeigt) genutzt werden können. Die bereits erwähnte direkte Markierung einzelner Aminosäuren ist hier am Beispiel einer Jodierung 105 der Seitenkette des Tyrosins gezeigt. Als bifunktionale Reportergruppen wurden hier p-SCN-Benzyl-DOTA 210 und p-Maleimid-Benzyl-DOTA 220 eingesetzt. Das gebräuchliche Kürzel DOTA steht hier für 1,4,7,10-Tetraazacyclododecan-1,4,7,10-tetraacetat. Diese Verbindung bildet stabile Komplexe, z.B. mit Lanthaniden und ist hinlänglich gut bioverträglich. Mit dem erfindungsgemäßen Baukasten 300 ließen sich so simultane Multiplex-Experimente für die Entwicklung neuer Kontrastmittel für medizinisch-diagnostische Anwendungen, beispielsweise bei der Kernspinresonanztomographie, durchführen. Die chemische Struktur des Metallbindenden Chelatbildners DOTA 110, 120 ist im rechten Teil der Figur schematisch gezeigt. Vorteilhafterweise kann der nicht-metallierte Chelatbildner mit unterschiedlichen Kationen 150 beladen werden. Die verwendeten unterschiedlichen Schraffuren der metallierten Chelatbildner stehen für Chelate zweier unterschiedlicher Metalle. Die Möglichkeit zur Einführung von mehr als nur zwei unterschiedlichen Metallspezies ist dem Fachmann an Hand der hier gegebenen Hinweise verständlich.

Das chelatisierte Metallion 150 ist typischerweise das Kation eines Lanthanoids, wie Lanthan, Cer, Praseodym, Neodym, Promethium, Samarium, Europium, Gadolinium, Terbium, Dysprosium, Holmium, Erbium, Thulium, Ytterbium oder Lutetium, beispielsweise La<3+>, Ce<3+>, Ce<4+>, Pr<3+>, Pr<4+>, Nd<3+>, Nd<4+>, Pm<3+>, Sm<2+>, Sm<3+>, Eu<2+>, Eu<3+>, Gd<3+>, Tb<3+>, Tb<4+>, Dy<3+>, Dy<4+>, Ho<3+>, Er<3+>, Tm<2+>, Tm<3+>, Yb<2+>, Yb<3+>, Lu<3+>.

An das Trägermaterial kann endständig entweder an die Aminogruppe 111 oder an die Carboxylgruppe 112 der entsprechenden endständigen Aminosäure ein reaktiver Linker angebracht sein, der nicht mit den Aminosäuren der Sequenz, wohl aber mit dem Target, wie beispielsweise dem Container 310 des modularen Baukastens 300 und/oder einem Biomolekül (einem Peptid, einem Polypeptid, einer Nukleinsäure, einem Protein - beispielsweise einem Enzym oder einem Antikörper - und/oder eine modifikationsspezifischen Spezies), reagieren kann oder der sich problemlos mit Hilfe eines Kopplungsreagenz mit dem genannten Target verknüpfen lässt.

Nach Bindung der Sequenz an das Target kann dann das Target direkt qualitativ oder quantitativ bestimmt werden, lässt sich mit entsprechenden Methoden durch seine Elementsignatur in einem biologischen System verfolgen, oder kann als Standard zur Kalibrierung oder Eichung eines Messgeräts oder zur Validierung einer Nachweismethode dienen.

Als Methoden zum Nachweis des Markermoleküls können jegliche für die Detektion der jeweiligen Heteroelemente geeignete analytische Messtechniken verwendet werden. Insbesondere werden atomspektroskopische Verfahren eingesetzt, wie z.B. Atomabsorptionsspektroskopie (AAS), Atomemissionsspektroskopie (AES), Atomfluoreszenzspektroskopie (AFS), Massenspektrometrie (MS), insbesondere Massenspektrometrie mit induktiv gekoppeltem Plasma (ICP-MS).

Eine besondere Bedeutung kommt der Markierung von Antikörpern durch den vorgeschlagenen Peptidmarker zu. Während in bisherigen Untersuchungen zur Biokonjugation die Zahl der signalgebenden Reportergruppen, die an ein Antikörpermolekül konjugiert werden konnte, mit typischerweise < 10 sehr begrenzt war, ermöglicht das vorgeschlagene Konzept des Einsatzes von Trägermolekülen einer definierten Sequenz und damit definierter Bindungseigenschaften sehr heterogene Konjugationen von einigen Zehn bis zu Hunderten von Reportergruppen je Antikörpermolekül.

Die Länge der Sequenz wird typischerweise so gewählt, dass sie die spezifischen Immuneigenschaften des Antikörpers nicht beeinträchtigt. Damit kann, beispielsweise in Abhängigkeit von der Affinität und Avidität des jeweiligen Antikörpers, die Sequenzlänge angepasst und die mit dem jeweiligen Antikörper erreichbare Nachweisempfindlichkeit optimiert werden. Vorteilhafterweise werden monoklonale Antikörper mit den hier beschriebenen Peptidmarkern dekoriert und für analytische Zwecke eingesetzt.

Anwendungen derartig markierter Antikörper, und damit auch des modularen Baukastens 300, liegen auf den Gebieten der Detektion einzelner Zellen, dem Nachweis von Analyten mittels ELISA, dem Nachweis von Proteinen oder dem Nachweis von Viren. Beispielsweise kann ein entsprechend markiertes Molekül ein Wirkstoff sein dessen Kinetik (Pharmakokinetik) untersucht werden soll. Ebenso kann ein beschriebenes Markermolekül zum spezifischen Nachweis von Proteinen in 2D-Elektrophorese oder Elektroblotting, insbesondere "Western-Blot"-Assay und im Immunoimaging verwendet werden.

Für die Bindung der Heteroelemente bestimmte reaktive Gruppen des Trägermaterials können reaktive Gruppen sein, wie sie typischerweise mit dem Begriff "bivalente Reportergruppen" beschrieben werden: In diesem Zusammenhang wird unter einer bivalenten Reportergruppe 320 eine reaktive heteroelementbindende Gruppe verstanden, die sowohl das Heteroelement binden als auch mit einer definierten Aminosäure reagieren kann. So kann beispielsweise ein Maleimid-Reaktand selektiv an Thiolgruppen (Cystein) und ein SCN-Reaktand selektiv an Aminogruppen (z.B. Lysin, Arginin) binden. Auch die terminale Aminogruppe eines Peptids kann mit einem SCN-Reaktanden mit einer Reportergruppe 320 dekoriert werden. Die heteroelementbindene reaktive Gruppe kann aus Chelat- und Komplexbildnern aufgebaut sein, die gezielt Metalle oder andere Markierungselemente binden. Als chelatbildende Gruppe kann beispielsweise DOTA (1,4,7,10-Tetraazacyclododecan-1,4,7,10-tetraacetat) oder DTPA (Diethylentriaminpentaacetat), sowie die bereits benannten chelatbildenden Gruppen verwendet werden.

Die beschriebene Markersubstanz, insbesondere die Reportergruppe, bzw. ein entsprechender Peptidmarker ist erforderlichenfalls hochgradig biokompatibel, da sein Grundgerüst vollständig aus natürlich vorkommenden Aminosäuren aufgebaut werden kann. Über die jeweilige Zusammensetzung der Aminosäuresequenz können die chemischen und physikalischen Eigenschaften wie z.B. Wasserlöslichkeit, Hydrophobizität, Restladung, isoelektrischer Punkt etc. gesteuert und so der jeweiligen Anwendung angepasst werden. Auch wenn in den Figuren nicht gezeigt, kann der Peptidmarker bzw. das Trägermolekül Aminosäuren aufweisen, die keine Reportergruppe 320 tragen und selbst auch nicht mit Heteroatomen markiert sind. Insbesondere derartige Aminosäuren bzw. Peptide aus derartigen Aminosäuren können zur elektrostatischen Stabilisierung oder zur Verbesserung der Löslichkeit der Markersubstanz eingesetzt werden.

Ein wesentlicher Vorteil der beschriebenen Markermoleküle gegenüber herkömmlichen Anwendungen ergibt sich aus der Homogenität automatisch synthetisierter Peptide und Polypeptide. Die beschriebene Markersubstanz umfasst ein Polymerbasismaterial, d.h. ein Peptid oder ein Polypeptid, einen oder mehrere verschiedene reaktive Linker und metallbindene Seitenketten (z.B. Chelatbildner). Da bisher bekannte Polymermaterialien äußerst heterogen bezüglich ihrer Kettenlänge sind, ist die Quantifizierung atomspektroskopischer Messdaten erschwert oder ganz unmöglich. Dieser Nachteil wird mit dem beschriebenen Trägermaterial überwunden. Zusätzlich werden wie beschrieben neue Anwendungsmöglichkeiten erschlossen.

Ein besonderer Vorteil der beschriebenen Reportergruppen und/oder Markersubstanzen besteht darin, dass eine hohe und frei einstellbare Nachweisempfindlichkeit erzielt werden kann, die molekulare Homogenität der zur Markierung verwendeten Moleküle, d.h. des Trägermaterials erheblich verbessert wird und eine Möglichkeit zur zusätzlichen Kodierung geschaffen wird, die multimodale Nachweistechniken unter Verwendung der molekularen Markierungen (engl.: molecular tags) ermöglicht.

Mit den hierin beschriebenen Reportergruppen und/oder Markermolekülen und den beschriebenen Verfahren und Verwendungen des modularen Baukastens 300 werden die Vorteile einer Metall-Chelatmarkierung zur Steigerung der Nachweisempfindlichkeit für unterschiedlichste Analyte erschlossen. Insbesondere kann über die beschriebene Metallchelat-Markierung von biospezifischen Liganden, biologisch aktiven Substanzen, Enzymen, Immunoglobulinen, insbesondere von monoklonalen Antikörpern, Antikörperfragmenten bzw. Fraktionen, die hohe Spezifität und Selektivität dieser Moleküle für unterschiedlichste molekulare und zelluläre Strukturen, wie z.B. Rezeptoren, Enzymsubstrate, Differenzierungsmarker (sogenannte cluster of differentiation; CD-Marker), Proteine, Glykolipide, Sphingolipide, Phospholipide, ja selbst für niedermolekulare (auch synthetische) Antigene wie Pestizide, Antibiotika oder pharmazeutische Wirkstoffe, mit der äußerst empfindlichen und wenig störanfälligen Atomspektroskopie kombiniert werden. Moderne atomspektroskopische Messtechniken und deren Kombination mit anderen Techniken, beispielsweise der Massenspektroskopie (MS, ICP-MS, ESI-/MALDI-MSMS, TOF-MS etc.) oder der Atomabsorptionsspektroskopie, der Röntgenfluoreszenzanalyse, EDX bieten die Möglichkeit verschiedene, nahe verwandte chemische Elemente selektiv aus ihrem Gemisch nachzuweisen.

Vorteilhafterweise können mit dem vorgeschlagenen Trägermaterial, insbesondere in Gestalt von Polypeptiden mit einer einzigen Markierung, d.h. mit Hilfe nur eines an das Target angekoppelten Markermoleküls, mehrere Heteroelemente mit dem markierten Molekül verknüpft werden. Das steigert einerseits die Nachweisempfindlichkeit für entsprechend markierte Biomoleküle erheblich, wenn zahlreiche Ionen eines Heteroelements eingeführt werden. Andererseits schafft das die beschriebenen Möglichkeiten einer Kodierung oder einer multimodalen Detektion, wenn mehrere verschiedene Heteroelemente in einem fest eingestellten Mengenverhältnis zueinander eingeführt werden.

Wenngleich hierin spezifische Ausführungsformen dargestellt und beschrieben worden sind, liegt es im Rahmen der vorliegenden Erfindung, die gezeigten Ausführungsformen geeignet zu modifizieren, ohne vom Schutzbereich der vorliegenden Erfindung abzuweichen. Die nachfolgenden Ansprüche stellen einen ersten, nicht bindenden Versuch dar, die Erfindung allgemein zu definieren.

## Patentansprüche

1. Modularer Baukasten zum Erkennen und/oder Identifizieren eines Ziels, zum Beispiel eines Zielmoleküls, einer Zielstruktur, oder des modularen Baukastens selbst, umfassend:
- einen Container (310); und
- eine Reportergruppe (320), die mit dem Container (310), vorzugsweise über einen ersten Linker (L1), verbunden ist, wobei die Reportergruppe (320) vorzugsweise konfiguriert ist, um den Container (310) identifizieren zu können;
wobei der modulare Baukasten (300) vorzugsweise weiterhin eine Erkennergruppe (330) umfasst, die mit dem Container (310) vorzugsweise über einen zweiten Linker (L2) verbunden ist, wobei die Erkennergruppe (330) vorzugsweise konfiguriert ist, um das Ziel, insbesondere das Zielmolekül oder die Zielstruktur, identifizieren zu können.

2. Modularer Baukasten nach Anspruch 1, umfassend mindestens eine Füllung (340), wobei die mindestens eine Füllung (340) sich innerhalb des Containers befindet und wobei typischerweise die mindestens eine Füllung (340) eine erste Füllung (342) aufweist, die mit dem Container (310) insbesondere über einen dritten Linker (L3) verbunden ist, und/oder wobei typischerweise die mindestens eine Füllung (340) eine zweite Füllung (344) aufweist, die nicht mit dem Container (310) verbunden ist.

3. Modularer Baukasten nach einem der vorstehenden Ansprüche, wobei der Container (310) ein Nanomaterial, wie beispielsweise ein Fulleren, ein Buckminster-Fulleren, einen Buckyball, eine C60-Struktur, ein Nanoröhrchen, ein Vesikel, ein Dendrimer und/oder ein Liposom aufweist, oder wobei der Container (310) ein Molekül ist, wie beispielsweise ein Chelatbildner, ein Peptid, ein Protein oder ein Antikörper.

4. Modularer Baukasten nach einem der vorstehenden Ansprüche, wobei die Reportergruppe (320) ein Atom ist, insbesondere ein Metall, das zur Detektion mittels Massenspektroskopie geeignet ist, wie beispielsweise eine seltene Erde, und/oder wobei die Reportergruppe (320) ein Molekül ist, das zur Detektion mittels oberflächenverstärkter Raman-Streuung und/oder zur organischen Massenspektroskopie geeignet ist, und/oder wobei die Reportergruppe (320) ein Farbstoff, insbesondere eine Fluoreszenzfarbstoff, ist, der zur Detektion mittels Mikroskopie geeignet ist.

5. Modularer Baukasten nach einem der vorstehenden Ansprüche, wobei die Erkennergruppe (330) ein chemisches Molekül aufweist, das eine hohe Affinität zu dem Zielmolekül oder der Zielstruktur aufweist, wobei die Erkennergruppe (330) typischerweise ein Antikörper, ein Biomolekül, ein Peptid, ein Polypetid, eine RNA, oder eine RNA aufweist.

6. Modularer Baukasten nach einem der vorstehenden Ansprüche, wobei die mindestens eine Füllung (340) einen Wirkstoff, einen Botenstoff, einen Inhibitor, einen Katalysator, eine radioaktive Substanz, die vorzugsweise konfiguriert ist, um das Zielmolekül oder die Zielstruktur zu zerstören und/oder bildgebend abzubilden, ein Kontrastmittel beispielsweise für Röntgen oder MRI, und/oder eine Reportergruppe aufweist.

7. Modularer Baukasten nach einem der vorstehenden Ansprüche, wobei der Container (310) ein Schloss (350) aufweist, mit dem der Container (310) geöffnet werden kann, um seinen Inhalt, insbesondere die mindestens eine Füllung (340) freizusetzen.

8. Verfahren zum Erkennen und/oder Identifizieren eines Ziels, zum Beispiel eines biologischen Zielmoleküls oder einer biologischen Zielstruktur, mit einem modularen Baukasten nach einem der Ansprüche 1 bis 7, der eine Erkennergruppe (330) aufweist, umfassend:
- Kontaktieren des Ziels mit der Erkennergruppe (330); und
- Detektieren des modularen Baukastens (300),
wobei der modulare Baukasten (300) vorzugsweise mindestens eine Füllung (340) umfasst, wobei das Verfahren vorzugsweise weiterhin das Freisetzen der mindestens einen Füllung (340) des Containers (310) des modularen Baukastens (300) umfasst.

9. Verfahren nach Anspruch 8, wobei das Verfahren zur Exploration von Drug Discovery Systemen für Multiplex-Experimente verwendet wird.

10. Verfahren zur Durchführung simultaner Multiplex-Experimente zur Entwicklung neuer Konstrastmittel durch einen Einsatz von Lanthaniden und/oder Metallen seltener Erden, unter Verwendung eines modularen Baukastens nach einem der Ansprüche 1 bis 7, wobei der Container (310) des modularen Baukastens (300) mindestens eine Füllung (340) aufweist, wobei die mindestens eine Füllung (340) ein oder mehrere Lanthanide und/oder ein oder mehrere Metalle seltener Erden aufweist, wobei das Verfahren umfasst:
- Kontaktieren des Ziels mit der Erkennergruppe (330);
- Freisetzen der mindestens einen Füllung (340) des Containers (310), insbesondere der Lanthanide und/oder Metalle seltener Erden; und
- Nachweis der Lanthanide und/oder Metalle seltener Erden mittels Laser Ablation ICP-MS, insbesondere deren Verteilung.

11. Modularer Baukasten gemäß einem der Ansprüche 1 bis 7 zur Verwendung in der Medizin.

12. Modularer Baukasten gemäß einem der Ansprüche 1 bis 7 zur Verwendung in der medizinischen Diagnostik.

13. Verwendung des modularen Baukastens gemäß einem der Ansprüche 1 bis 7 bei der Qualitätskontrolle bei der Herstellung von pharmazeutischen Mitteln, bei der Nachverfolgung der Transportwege von pharmazeutischen Mitteln, und/oder bei der Bestimmung der Verteilung von pharmazeutischen Mitteln in einem biologischen oder medizinischen Objekt, beispielsweise in einem Organismus.

14. Verwendung des modularen Baukastens gemäß einem der Ansprüche 1 bis 7 zur Herstellung eines Systems zur Verwendung in der Medizin, beispielsweise zur Verwendung in der medizinischen Diagnostik.

15. Verwendung des modularen Baukastens gemäß einem der Ansprüche 1 bis 7 zur Herstellung eines Systems zur Verwendung gemäß Anspruch 13.
